(19) Europäisches Patentamt
European Patent Office
Office européen des brevets

(11) **EP 3 869 512 B1**

(12) **EUROPEAN PATENT SPECIFICATION**

(45) Date of publication and mention
of the grant of the patent:
**12.11.2025 Bulletin 2025/46**

(21) Application number: **20216792.0**

(22) Date of filing: **23.12.2020**

(51) International Patent Classification (IPC):
**G16C 20/10** (2019.01)   **G16C 20/30** (2019.01)
**A61N 1/32** (2006.01)

(52) Cooperative Patent Classification (CPC):
**G16C 20/30; A61N 1/327; G16C 20/10**

(54) **IN-SILICO DESIGN OF ELECTROPORATION EXPERIMENTS FOR TOPICAL AND TRANSDERMAL DRUG DELIVERY APPLICATIONS**

IN-SILICO-ENTWURF VON ELEKTROPORATIONSVERSUCHEN FÜR TOPISCHE UND TRANSDERMALE ARZNEIMITTELABGABEANWENDUNGEN

CONCEPTION IN-SILICO D'EXPÉRIENCES D'ÉLECTROPORATION POUR DES APPLICATIONS D'ADMINISTRATION DE MÉDICAMENTS TOPIQUE ET TRANSDERMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.02.2020 IN 202021007335**

(43) Date of publication of application:
**25.08.2021 Bulletin 2021/34**

(73) Proprietor: **Tata Consultancy Services Limited Maharashtra (IN)**

(72) Inventors:
• **Gajula, Kishore**
411013 Pune - Maharashtra (IN)
• **Gupta, Rakesh**
411013 Pune - Maharashtra (IN)
• **Rai, Beena**
411013 Pune - Maharashtra (IN)

(74) Representative: **Goddar, Heinz J.**
**Boehmert & Boehmert**
**Anwaltspartnerschaft mbB**
**Pettenkoferstrasse 22**
**80336 München (DE)**

(56) References cited:
• GAJULA KISHORE ET AL: "Multiscale Modeling of Skin Electroporation", LANGMUIR, vol. 36, no. 24, 1 June 2020 (2020-06-01), US, pages 6651 - 6660, XP055809796, ISSN: 0743-7463, DOI: 10.1021/acs.langmuir.0c00500

• KISHORE GAJULA ET AL: "In-Silico Skin Model: A Multiscale Simulation Study of Drug Transport", JOURNAL OF CHEMICAL INFORMATION AND MODELING, vol. 57, no. 8, 18 July 2017 (2017-07-18), US, pages 2027 - 2034, XP055523481, ISSN: 1549-9596, DOI: 10.1021/acs.jcim.7b00224

• GUPTA RAKESH ET AL: "Electroporation of Skin Stratum Corneum Lipid Bilayer and Molecular Mechanism of Drug Transport: A Molecular Dynamics Study", LANGMUIR, vol. 34, no. 20, 30 April 2018 (2018-04-30), US, pages 5860 - 5870, XP055810115, ISSN: 0743-7463, DOI: 10.1021/acs.langmuir.8b00423

• BECKER S M ET AL: "Thermal in vivo skin electroporation pore development and charged macromolecule transdermal delivery: A numerical study of the influence of chemically enhanced lower lipid phase transition temperatures", INTERNATIONAL JOURNAL OF HEAT AND MASS TRANSFER, ELSEVIER, AMSTERDAM, NL, vol. 51, no. 7, 30 July 2007 (2007-07-30), pages 2060 - 2074, XP085298659, ISSN: 0017-9310, DOI: 10.1016/J.IJHEATMASSTRANSFER.2007.06.010

• FRASCH H FREDERICK ET AL: "Application of numerical methods for diffusion-based modeling of skin permeation", ADVANCED DRUG DELIVERY REVIEWS, ELSEVIER, AMSTERDAM , NL, vol. 65, no. 2, 10 January 2012 (2012-01-10), pages 208 - 220, XP028973390, ISSN: 0169-409X, DOI: 10.1016/J.ADDR.2012.01.001

# EP 3 869 512 B1

**Description**

TECHNICAL FIELD

The disclosure herein generally relates to topical and transdermal drug delivery applications, and, more particularly, to systems and methods for in-silico design of electroporation experiments for topical and transdermal drug delivery applications.

BACKGROUND

Human skin, the largest external organ of the body, provides opportunities for numerous topical healthcare and personal care applications. However, leveraging abundance of skin is challenging due to barrier provided by the outermost layer of the skin's epidermis known as stratum corneum (SC). This layer is made up of corneocytes (having proteins and natural moisturizing factor) and lipid matrix, where the latter fills an extracellular space of former. This unique arrangement provides a barrier for permeation of molecules across SC. The lipid matrix is made up of three class of molecules namely ceramides, cholesterol and free fatty acids. Among these, ceramides have further various sub classes based on their head group structure and lipid chain length, whereas free fatty acid only have varying chain length. As per study, till date, 18 different types of ceramides have been found in human skin.

Transdermal and topical drug delivery have several advantages over oral and injection route such as avoiding gastro-intestinal and first-pass liver degradation, safe and convenient for administration at steady or time varying delivery rates, to name a few. Despite this, its application is only limited to small and mostly lipophilic molecules. Furthermore, the lag time to reach steady state flux even for these molecules remains in the range of hours during passive diffusion conditions. In order to deliver therapeutics (hydrophilic drugs, big proteins etc.) in the deeper layer of skin, barrier provided by SC has to be breached. Currently, two specific methods namely, a) passive methods such as use of permeation enhancers (ethanol, ionic liquids, nanomaterials, biomaterials, etc.) and b) active methods such as electroporation, iontophoresis and sonophoresis are being used. Several researchers have reported use of passive methods for enhancing of drug permeation through skin. However, these conventional research works have their own limitations. For instance, these lack in providing a molecular level resolution and model of realistic skin lipid matrix and thus lead to less accuracy in terms of providing results.

Gajula Kishore et al.: "In-Silico Skin Model: A Multiscale Simulation Study of Drug Transport" teaches a multiscale modeling framework to obtain the release profile of three drugs, namely, caffeine, fentanyl, and naphthol, through skin stratum corneum (SC). The diffusion coefficients of drugs in the SC lipid matrix were determined from multiple constrained molecular dynamics simulations. The calculated diffusion coefficients were then used in the macroscopic models to predict the release profiles of drugs through the SC. The reported multiscale modeling framework would provide insight into the delivery mechanisms of the drugs through the skin and shall act as a guiding tool in performing targeted experiments to come up with a suitable delivery system.

Rakesh Gupta et al.: "Electroporation of Skin Stratum Corneum Lipid Bilayer and Molecular Mechanism of Drug Transport: A Molecular Dynamics Study", teaches that extensive atomistic molecular dynamics simulation of skin lipids has been performed at various external electric fields. This document further discloses for the first time the pore formation in the skin lipid bilayer during electroporation and the effect of the applied external electrical field on the pore formation dynamics in the lipid bilayer of different sizes and compositions. Further, this document discloses that the molecular-level understanding obtained could help in optimizing/designing the electroporation experiments for effective drug delivery. For a given skin composition and size of the drug molecule, the combination of pore formation time and pore growth model can be used to know a priori the desired electric field and time for the application of the electric field.

S. M. Becker et al.: "Thermal in vivo skin electroporation pore development and charged macromolecule transdermal delivery: A numerical study of the influence of chemically enhanced lower lipid phase transition temperatures" teaches the benefits of the combination of the chemical enhancer, terpene d-limonene with low voltage electroporation pulses in order to further aid in electroporation pore development resulting from fluidization of the lipid structures within the stratum corneum. A transient finite volume model is developed in which the thermal and electrical behavior associated with electroporation of in vivo human skin is analyzed and lipid phase transition is represented by a melting process. The Nernst-Planck model is used to represent the electrophoretic-assisted transport of large charged molecules through the skin.

SUMMARY

Embodiments of the present disclosure present technological improvements as solutions to one or more of the above-mentioned technical problems recognized by the inventors in conventional systems. For example, in one embodiment, there is provided a processor implemented method for in-silico design of electroporation experiments for topical and transdermal drug delivery applications. In another aspect, there is provided a system for in-silico design of electroporation experiments for topical and transdermal drug delivery applications. In yet another aspect, there are provided one or more non-transitory machine-readable information storage mediums comprising one or more instructions which when executed by one or more hardware processors cause in-silico design of electroporation experiments for topical and transdermal drug delivery applications. The invention is defined in the appended set of claims.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts an exemplary block diagram of a system for in-silico design of electroporation parameters for topical and transdermal drug delivery applications, in accordance with an embodiment of the present disclosure.

FIG. 2 depicts an exemplary flow chart for in-silico design of electroporation parameters for topical and transdermal drug delivery applications using the system of FIG. 1, in accordance with an embodiment of the present disclosure.

FIG. 3 depicts a molecular electroporation model of stratum corneum (SC) layer of human skin, in accordance with an example embodiment of the present disclosure.

FIG. 4 depicts electric field being applied across lipid bilayer of the stratum corneum layer of human skin and pore formation and its growth, in accordance with an example embodiment.

FIG. 5 depicts electric field of 0.9 volts per nanometer (V/nm) and 0.25 V/nm being applied across the lipid bilayer during electroporation simulation in accordance with an example embodiment of the present disclosure.

FIG. 6 depicts introduction of drug molecule in a stable pore, in accordance with an example embodiment of the present disclosure.

FIG. 7A depicts a graphical representation of change in flux of fentanyl for different thickness of the SC layer during passive diffusion, in accordance with an example embodiment of the present disclosure.

FIG. 7B depicts a graphical representation of change in cumulative release of fentanyl for different thickness of the SC layer during the passive diffusion, in accordance with an example embodiment of the present disclosure.

FIG. 8A depicts a graphical representation illustrating a comparison of experimental measured fentanyl flux across skin during electroporation versus flux calculated using a known multiscale model using two different diffusion coefficients from molecular dynamics (MD) simulations, in accordance with an embodiment of the present disclosure.

FIG. 8B depicts a graphical representation illustrating a comparison of experimental measured cumulative release profile of fentanyl flux across skin during electroporation versus cumulative release profiles of fentanyl calculated using the previous multiscale model using two different diffusion coefficients from the MD simulations, in accordance with an embodiment of the present disclosure.

FIG. 9A depicts a graphical representation illustrating a comparison of experimental measured fentanyl flux across skin during electroporation versus calculated fentanyl flux using multiscale model during passive conditions and during electroporation, in accordance with an example embodiment of the present disclosure.

FIG. 9B depicts a graphical representation illustrating a comparison of experimental measured cumulative release profile of fentanyl flux across skin during electroporation versus calculated cumulative release using multiscale model during passive conditions and during electroporation, in accordance with an example embodiment of the present disclosure.

FIGS. 10A through 11F depict fentanyl flux profiles for factorial simulations, in accordance with an example embodiment of the present disclosure.

FIGS. 12A through 12D depict various cumulative release profiles for factorial simulations, in accordance with an example embodiment of the present disclosure.

FIGS. 13A-13B depict a comparative study of cumulative release at 6hrs and steady state flux for different voltages and different pulse durations, in accordance with an example embodiment of the present disclosure.

FIG. 14 depicts a design space for selecting an electroporation protocol for a specific application, in accordance with an example embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts.

As mentioned above, human skin is the largest external organ of the body and provides a selective barrier to therapeutics applied topically. Molecules having specific chemical and physical properties can only penetrate the deeper layer of the human skin. However, the lag time for reaching a steady state in the deeper layer is generally of the order of hours. In order to deliver higher molecular weight, charged and hydrophilic therapeutics in the deeper layer, skin barrier must be breached out. Electroporation is one of the methods used to breach the skin barrier for enhancement of drug permeation and reduction of lag time. However, underlying mechanism responsible for enhancement of drug permeation is not well understood.

There are several in-vivo and in-vitro experimental studies that have been reported on skin electroporation. For instance, Vanbever et al. (Transdermal delivery of metoprolol by electroporation. Pharm. Res. 1994, 11, 1657-1662) performed electroporation experiments on rat skin and reported that cumulative permeation of metoprolol drug changed with pulse condition. Cumulative release increased with increase in voltage, pulse duration and number of pulses when varied individually. Prausnitz et al. (Transdermal transport efficiency during skin electroporation and iontophoresis. J. Controlled Release 1996, 38, 205-217) studied the transport of calcein through human epidermis during electroporation and iontophoresis under various pulse conditions. Efficacies for both iontophoresis and electroporation increased with increase in voltage and current. There was no dependency of pulse length, rate, waveform energy and total charge delivered on efficacies. Zewert et al. (Creation of transdermal pathways for macromolecule transport by skin electroporation and a low toxicity, pathway-enlarging molecule. Bioelectrochem. Bioenerg. 1999, 49, 11-20) studied the hypothesis of formation of aqueous pathways through SC via in-vitro experiments. High voltage pulse electroporates only the lipid matrix, however the introduction of sodium thiosulfate during electroporation weakened keratin matrix in the corneocytes thereby providing pathways for transport of molecules with higher molecular weight. Recently, through atomistic molecular simulation it was shown that on application of certain threshold electric field the pores are created in the skin lipid layer. The size of pores increased with increase in the applied electric field. The drug diffusion through these pores also increased, however it could not still explain why the drug permeation flux increased several folds on the application of electric field.

Further, there are some models reported in the literature describing skin electroporation at macroscopic level. For example, Becker et al. (Thermal in vivo skin electroporation pore development and charged macromolecule transdermal delivery: a numerical study of the influence of chemically enhanced lower lipid phase transition temperatures. Int. J. Heat Mass Transfer 2008, 51, 2060-2074) developed a transient skin electroporation model for thermal pore development and solute transport. The transdermal transport of solute was greatly increased with combining enhancer (terpene) during electroporation. Pavšelj et al. (A numerical model of skin electropermeabilization based on in vivo experiments. Ann. Biomed. Eng. 2007, 35, 2138-2144) developed a theoretical model based on finite element method to study skin electropermeabilization with electrical and thermal effects. Increase in pulse amplitude resulted in higher electric field in the tissues. These models are not multiscale in nature and are limited to permeation time of the order of milliseconds. Dermol-Černe et al. (From cell to tissue properties-modeling skin electroporation with pore and local transport region formation. IEEE Trans. Biomed. Eng. 2018, 65, 458-468) developed skin electroporation model connecting at different levels (cell membrane to skin layer to skin tissue containing several layers). Pore formation in cell membranes and local transport region (LTR) formation in SC were also studied. Density of LTRs increased during short high-voltage pulses and size of LTRs increased during low voltage long pulses due to joule heating and lipid melting around defects.

Therefore, developing a model to study the transport of drugs through skin during electroporation under realistic experimental conditions is necessary. A multiscale model for transport of drugs through SC under passive diffusion conditions has also been reported (e.g., refer India patent application: IN201721007631 titled "METHOD AND SYSTEM FOR IN-SILICO TESTING OF ACTIVES ON HUMAN SKIN" Filed on March 3, 2017), wherein diffusion coefficients of drugs fentanyl, caffeine and napthol were obtained using molecular dynamics (MD) simulations. The cumulative release of drugs through SC were obtained using finite element method simulations. The present disclosure is an extension of previously built multiscale model of skin SC to study the passive diffusion of drugs.

More specifically, in the present disclosure, multiscale model of skin electroporation is provided by way of various embodiment that implements a multiscale modeling technique wherein molecular phenomena are connected to macroscopic model. At atomic scale, molecular dynamics simulations of lipid matrix of human stratum corneum have been performed under the influence of external electric field. The pores get formed during the electroporation process and the transport properties (diffusivity) of drug molecules are computed. The diffusion coefficient obtained during electroporation was found to be higher than the passive diffusion. However, this alone could not explain the multifold increase in the drug flux on application of electric field as observed in the experiments being performed by the embodiments of the present disclosure. Hence, a finite element method/analysis (FEM/FEA) model of skin stratum corneum has also been developed by the present disclosure. The release of drug molecule (e.g., fentanyl utilized) through this model is compared with available experimental results. Both experimental and simulated results of many folds increase in drug flux and release profiles are comparable.

Once validated, the systems and methods of the present disclosure was used for design of experiments study by exploring the electric pulse parameters such as voltage, pulse duration and number of pulses. In other words, pulse parameters (also referred as pre-defined pulse parameters) include pulse voltage, pulse rate, pulse duration, pulse shape and the like. Voltage and pulse duration were varied for a fixed number of square pulses and flux, cumulative release of fentanyl was obtained. This multiscale modelling framework (also referred as 'multiscale modelling technique' or 'multiscale analysis' in the present disclosure and interchangeably used hereinafter) provides valuable insight at molecular and macroscopic level to design the electroporation experiments. The framework can be utilized as a design tool for selection of electroporation protocols, which may be interest for a specific application.

The above description and method of the present disclosure may be better understood by a person having ordinary skill in the art or person skilled in the art by way of following example. More specifically, the present disclosure has studied the conventional research works and has further focused on active method such as electroporation. In the present disclosure, during electroporation, a high voltage is applied across the skin for nano to millisecond time scales. It is hypothesized that due to high electric field, pores are formed in the skin SC, which enable the transport of drug molecules (also referred as 'molecules' and interchangeably used hereinafter) across the human skin (also referred as 'skin' and interchangeably used hereinafter). In other words, molecular dynamics simulations of skin SC lipid layer are performed under (external) electric field. The diffusion coefficients are calculated in passive and active (with electroporation) conditions. Further, macroscopic electroporation model is developed and Nernst-Planck equation and Laplacian equation for voltage have been solved to predict flux and cumulative release of the drug molecules (e.g., fentanyl considered as a drug molecule and case study). The effect of skin SC thickness and diffusion coefficients of fentanyl on flux and cumulative release is presented by way of various experiments and the same is depicted in various representations by embodiments of the present disclosure. Finally, an integrated multiscale model of skin electroporation is developed to study the mechanism at different length and time scales. The model was further tested against the available experimental data on fentanyl permeation under the external electric field. The model was further utilized as design tool in designing electroporation experiments. Full factorial design simulations were performed for four different voltages and three pulse durations for given number of pulses (discussed in the later section of the present disclosure).

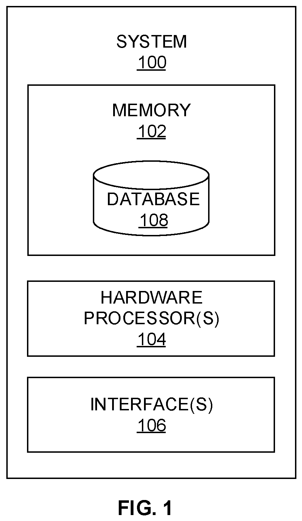

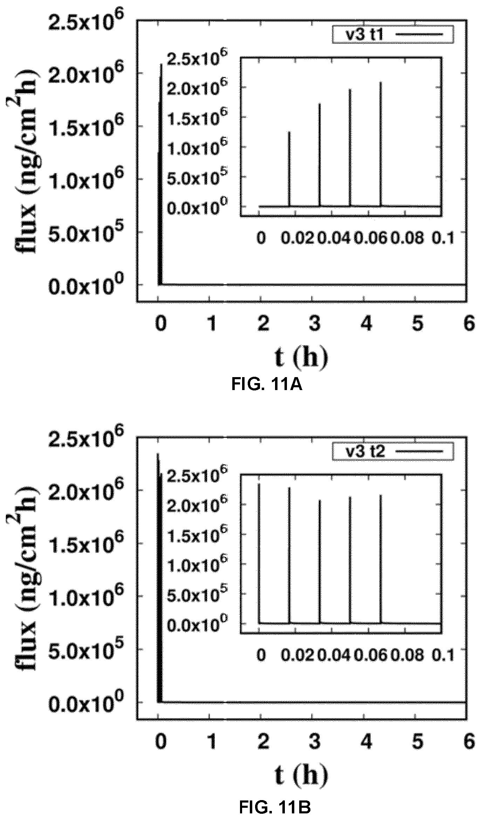

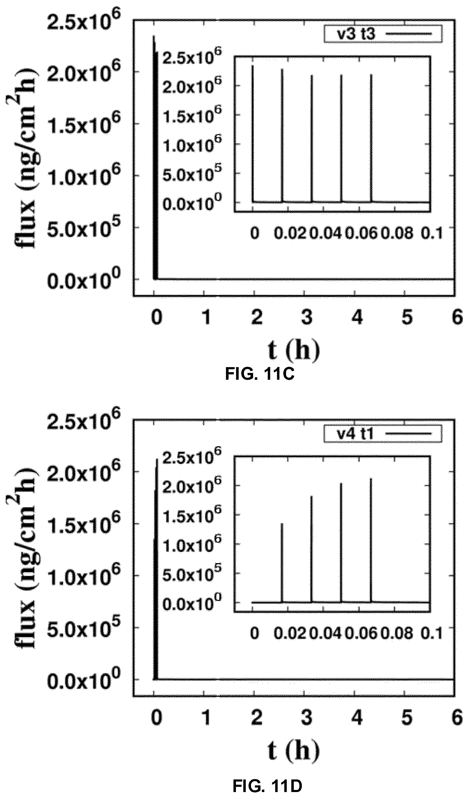

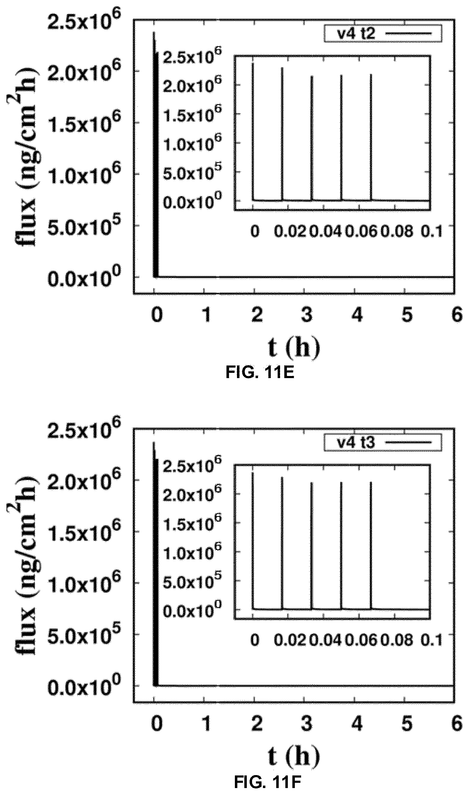

Referring now to the drawings, and more particularly to FIGS. 1 through 14, where similar reference characters denote corresponding features consistently throughout the figures, there are shown preferred embodiments and these embodiments are described in the context of the following exemplary system and/or method.

FIG. 1 depicts an exemplary block diagram of a system 100 for in-silico design of electroporation parameters for topical and transdermal drug delivery applications, in accordance with an embodiment of the present disclosure. The system 100 may also be referred as 'multiscale modeling system', 'multiscale analysis system' and may be interchangeably used hereinafter. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information, for example, molecular electroporation model of stratum corneum layer of human skin, pore formation, stabilized pore information, one or more diffusion coefficients of one or more drug molecules through the stable pore, skin macroscopic structure of the stratum corneum layer of the human skin, concentration gradient of the one or more drug molecules, flux profile and cumulative release profile of the one or more drug molecules being calculated using the concentration gradient, and the like.

The information stored in the database 108 may further comprise information on (i) one or more pre-defined boundary conditions of the macroscopic structure of the stratum corneum layer, (ii) one or more pre-defined pulse parameters and (ii) one or more properties obtained from the molecular electroporation model, fine-tuned pulse parameters based on the calculated flux profile and the calculated cumulative release profile, and the like. The database further comprises information pertaining to the one or more pre-defined pulse parameters such as pulse duration, a pulse type (e.g., exponential decay, rectangle pulse, bell type), and an applied voltage.

In an embodiment, the memory 102 may store (or stores) one of more techniques. For instance, molecular dynamics simulation technique(s), Finite Element Method/Analysis technique(s) and the like may be comprised in the memory 102 and executed accordingly to perform one or more methodologies described hereinafter. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

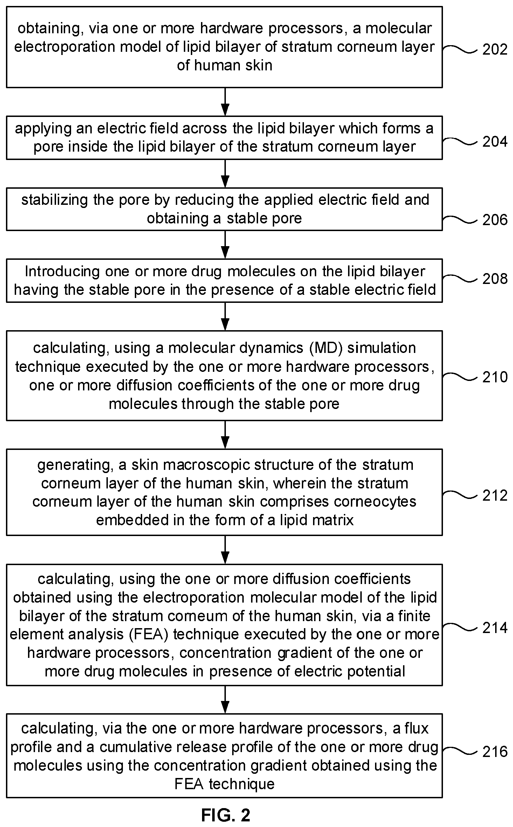

FIG. 2, with reference to FIG. 1, depicts an exemplary flow chart for in-silico design of electroporation parameters for topical and transdermal drug delivery applications using the system 100 of FIG. 1, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104.

Human SC layer comprises of 17-25 layers of flat corneocytes, which are interconnected by complex multilayer lipid lamella structure. The corneocytes and lipid lamella are arranged in brick and mortar assembly, respectively. The corneocytes are hydrophilic as compared to lipid lamella, which is highly hydrophobic in nature. Molecules having specific chemical properties can pass through SC layer. Even if molecules partition over corneocytes, ultimately, they must travel through tortuous lipid lamella. Hence, the skin barrier function is mostly determined by the tight packing of lipid lamella.

The lipid matrix is made up of three class of lipids, namely ceramides, cholesterol (and its derivatives) and free fatty acids in certain ratio. This lipid matrix is complex in nature and may contain hundreds of different types of ceramides and free fatty acids based on location of body part. Simulating, a lipid layer system incorporating each of these lipids is beyond with decent computing capability. However, in order to simulate a realistic skin composition, a lipid layer having one representative molecule from each lipid class was used by the present disclosure. The N-acylated sphingosine ceramide (CER), cholesterol (CHOL) and palmitic acid (FFA) is used to represent each class. Previously, this model was used to predict the permeability of molecules through SC, the flux of drug molecules through SC and understanding of skin electroporation mechanism at molecule scale. Here, the same model is used to mimic the SC lipid layer.

The CER was modelled using combination of GROMOS and Berger force field. The parameters for CHOL, FFA and fentanyl were taken from earlier studies (e.g., refer India patent application: IN201721007631 titled "METHOD AND SYSTEM FOR IN-SILICO TESTING OF ACTIVES ON HUMAN SKIN" Filed on March 3, 2017). The methyl group of CER and FFA chains were treated as united atom (CH3 and CH2 considered as C with increased molecular weight) with zero charge. Dihedrals of the hydrocarbon chains of the CER and FFA were represented with Ryckaert-Bellemans potential. The headgroup atoms (even H) of each lipid are represented explicitly in order to capture the polar effect. The charges on headgroup atoms were taken from earlier studies. One important thing to note is that the CHARMM force field reproduces experimental properties of CER bilayer better than the force field used here, but only at high temperature. At physiological temperature, both force fields give similar results. Since CHARMM force field is computationally expensive as compared to combination of Berger and GROMOS, the latter one was used in the present disclosure and implemented accordingly.

Each simulation was carried out in NPT ensemble using the GROMACS molecular dynamics package. The temperature was controlled at a temperature of ~ 310 K, using Nose-Hover thermostat with a time constant of 2 ps. Pressure was controlled by Parrinello-Rahman barostat with a time constant of 12 ps and compressibility of 4.0 x 10-5 bar-1 with semi-isotropic coupling. The pressure was controlled in X, Y and Z direction independently in order to obtain the tension free bilayer. The bonds of lipid molecules and water were constrained using LINCS and SETTLE algorithm, respectively. Each simulation was performed with 2 fs time step. A cut-off of 1.2 nm was used for Van der Waals and electrostatic interactions. Long-range electrostatic interactions were computed using particle mesh Ewald (PME) method. The bilayer systems were periodic in all three directions. The neighbor list was updated at every 10 steps. The configuration was sampled at every 10 ps.

The bilayer had 208 CER, 200 CHOL, 208 FFA and 24640 water molecules. The initial size of bilayer system was 9.79 nm x 9.79 nm x 11.61 nm. The bilayer was equilibrated for 200 ns in NPT ensemble as per the above protocol.

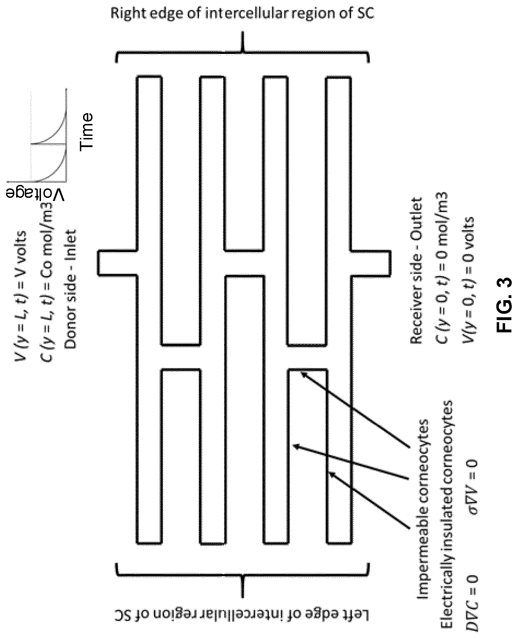

Referring to FIG. 2, the steps of the method of the present disclosure will now be explained with reference to above explanation/introduction, components of the system 100 of FIG. 1, the flow diagram as depicted in FIG. 2, and FIGS. 3 through 14. In an embodiment, at step 202 of the present disclosure, the one or more hardware processors 104 obtain a molecular electroporation model of stratum corneum layer of human skin, wherein the stratum corneum layer comprises a lipid bilayer. FIG. 3, with reference to FIGS. 1 and 2, depicts the molecular electroporation model of stratum corneum layer of the human skin, in accordance with an example embodiment of the present disclosure.

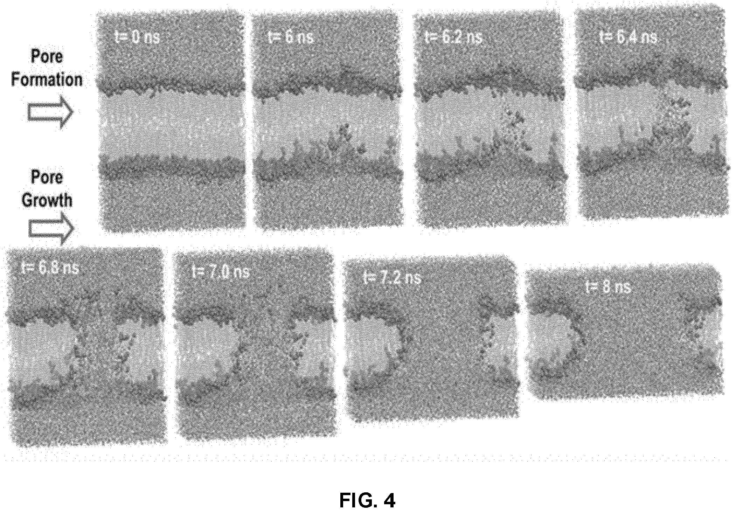

In an embodiment, at step 204 of the present disclosure, the one or more hardware processors 104 apply an electric field across the lipid bilayer which forms a pore inside the lipid bilayer of the stratum corneum layer. FIG. 4, with reference to FIGS. 1 through 3, depicts electric field being applied across the lipid bilayer and pore formation and its growth, in accordance with an example embodiment. More specifically, FIG. 4 shows a front view of the lipid bilayer during electroporation simulation at applied electric field of 0.9 V/nm. The potential is positive at the top leaflet of the lipid bilayer relative to the bottom leaflet. In FIG. 4, top row snapshots show the pore formation process in the lipid layer during electroporation. Bottom row snapshots shows growth of the pore after it connects both the leaflets.

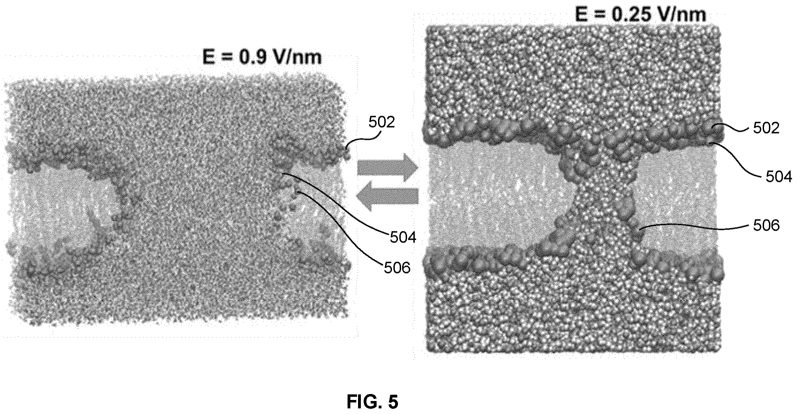

FIG. 5, with reference to FIGS. 1 through 4, depicts electric field of 0.9 volts per nanometer (V/nm) and 0.25 V/nm being applied across the lipid bilayer during electroporation simulation in accordance with an example embodiment of the present disclosure. As can be seen, the chains of lipid molecules are in transparent mode for the purpose of clarity. Details of N-acylated sphingosine ceramide (CER) represented as 502, cholesterol (CHOL) represented as 504 and palmitic acid (FFA) represented as 506 are shown accordingly. The potential is positive at the top leaflet of the bilayer relative to the bottom leaflet. Four independent systems were simulated at 0.25 V/nm and snapshots of the system, in which pore remained stable, is shown here.

In an embodiment, at step 206 of the present disclosure, the one or more hardware processors 104 stabilize the pore by reducing the applied electric field (e.g., stable electric field) to obtain a stable pore. Pore stabilization is depicted in FIG. 4.

In an embodiment, at step 208 of the present disclosure, the one or more hardware processors 104 introduce one or more drug molecules on the lipid bilayer having the stable pore under the electric field. In the present disclosure, drug molecule fentanyl has been considered. In the present disclosure, the lipid bilayer, which had stable pore, was further used for fentanyl permeation simulation under the electric field. Three fentanyl drug molecules were inserted in the upper water layer of the stable pore bilayer system. Four independent system, having drug molecules at different XY positions, were created. The water molecules, overlapping with inserted drug molecules, were removed. Each system was energy minimized and further subjected to NPT MD run for 25 ns at electric field of 0.25 V/nm.

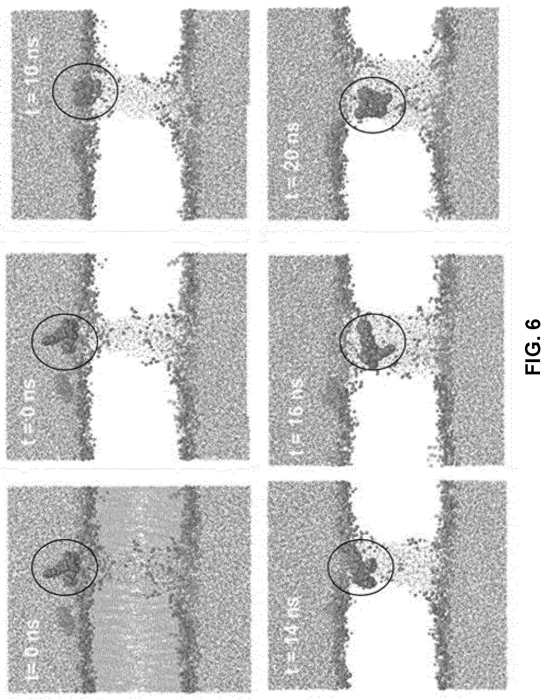

FIG. 6, with reference to FIGS. 1 through 5, depicts introduction of drug molecule in the stable pore, in accordance with an example embodiment of the present disclosure. More specifically, FIG. 6 depicts the lipid bilayer during the simulation at electric field of 0.25 V/nm in presence of fentanyl molecule(s). The chains of lipid molecules (in transparent style) are shown in one snapshot only for the purpose of clarity. The CER, CHOL, FFA and drug molecules are shown accordingly in FIG. 6. Further, FIG. 6 depicts encircled shape that represents fentanyl molecule(s). The time given in each snapshot, represent the kinetics of permeation of the fentanyl molecule through the pore of bilayer. The permeation process is stochastic in nature and exact value of time represented here may not be exactly reproducible. Three independent simulations were run and snapshots of one of them is depicted in FIG. 6.

In an embodiment, at step 210 of the present disclosure, the one or more hardware processors 104 calculate, using a molecular dynamics (MD) simulation technique executed by the one or more hardware processors, one or more diffusion coefficients of the one or more drug molecules through the stable pore. In one example embodiment of the present disclosure, the one or more diffusion coefficients comprise active diffusion coefficient(s) and/or passive diffusion coefficient(s). In the present disclosure, active diffusion coefficient refers to a diffusion coefficient obtained in presence of electric field and Passive diffusion coefficient refers to a diffusion coefficient obtained in absence of electric field.

In an embodiment, at step 212 of the present disclosure, the one or more hardware processors 104 generate a skin macroscopic structure of the stratum corneum layer of the human skin, wherein the stratum corneum layer of the human skin comprises corneocytes embedded in the form of a lipid matrix.

In an embodiment, at step 214 of the present disclosure, the one or more hardware processors 104 calculate, using the one or more diffusion coefficients obtained using the electroporation molecular model of the lipid bilayer of the stratum corneum of the human skin, via a finite element analysis technique executed by the one or more hardware processors, concentration gradient of the one or more drug molecules in presence of electric potential, In an embodiment, at step 216 of the present disclosure, the one or more hardware processors 104 calculate a flux profile and a cumulative release profile of the one or more drug molecules using the concentration gradient via the FEA technique.

The steps 214 and 216 are better understood by way of following description: In step 214, the concentration gradient was obtained by solving a Laplacian equation and Nernst-Planck equations in lipid regions of the stratum corneum layer respectively. The Laplacian equation and Nernst-Planck equations are solved based on (i) one or more pre-defined boundary conditions of the macroscopic structure of the stratum corneum layer along with one or more pre-defined pulse parameters and (ii) one or more properties obtained from the molecular electroporation model. Fick's second law governs the passive diffusion of drug through SC layer. In the presence of electric field, transport of drug molecules is given by Nernst-Planck equation. Electrophoretic term is added to Fick's second law to arrive at this equation. The electric potential (voltage) across SC is solved using Laplacian equation, which is given by

$$\nabla.\left(\sigma \nabla V\right) = 0 \qquad (1)$$

Nernst-Planck equation for drug transport in the presence of electric field is given by

$$\frac{\partial C}{\partial t} = \nabla.\left[\left(D\nabla C\right) + \left(mC\nabla V\right)\right] \qquad (2)$$

where, $C$ = concentration of permeating drug molecule, $D$ = effective diffusion coefficient of drug molecule in SC lipid region, $V$ = voltage applied across the skin, $\sigma$ = electrical conductivity and $m$ = electrophoretic mobility

The geometry of intercellular regions of SC layer (shown in FIG. 3) was built using a tool of COMSOL. The geometry was meshed with quadrilateral elements. Both governing equations 1 and 2 were solved using finite element method in COMSOL Multiphysics software. The following modules of COMSOL were used in developing the macroscopic model. Transport of diluted species module was used to solve the Nernst-Planck equation, electro currents module was used to solve the Laplacian equation for potential and events feature to capture discontinuity in voltage boundary condition. Multifrontal Massively Parallel Sparse direct solver was used in the simulations.

The inlet (donor side of SC) was maintained at constant concentration of Co mol/m$^3$. Co was calculated as product of concentration in donor solution (vehicle) and partition coefficient between SC and vehicle. The outlet (receiver side of SC) was maintained at 0 mol/m$^3$. The corneocytes were assumed to be impermeable and no flux boundary condition was applied around edges of corneocytes. Voltage difference of V volts was applied across SC layer. The inlet was maintained at voltage of V volts and outlet was maintained at 0 volts. Exponential decay pulses were introduced at the inlet and expression for voltage is given by the equation below.

$$V(t) = V_o(t)\exp\left(-\frac{t}{t_o}\right) \qquad (3)$$

where, $V_o(t)$ is voltage applied across SC layer, $t$ = time during electroporation, $t_o$ = pulse duration during electroporation.

The corneocytes were assumed to be insignificantly affected by high voltage pulse compared to lipids. The corneocytes were electrically insulated during electroporation. The total time of studying transport was divided into electroporation time (15 exponential decay pulses) and passive diffusion time (after electric field is withdrawn). The passive diffusion coefficient ($D_{passive}$) of fentanyl was obtained using MD simulations. The active diffusion coefficient ($D_{active}$) of fentanyl through aqueous pore formed during electroporation was obtained from the MD simulations. The electrophoretic mobility of fentanyl under influence of electric field was calculated using Einstein-Smoluchowski relation.

$$m = \frac{z*D*F}{R*T} \qquad (4)$$

where, $D$ is the diffusion coefficient of a drug molecule through SC, $z$ is charge of drug molecule, $F$ is faraday's constant, $R$ is gas constant and $T$ is the temperature.

The partition coefficient between SC and donor solution (vehicle) was calculated from Potts and Guy correlation.

$$K_{SC/vehicle} = (K_{o/w})^{0.71} \qquad (5)$$

where, $K_{SC/vehicle}$ is partition coefficient between SC layer and vehicle, $K_{o/w}$ is octanol/water partition coefficient.

The octanol/ water partition coefficient at pH 4.0 was extrapolated using available literature values at different pH values. The octanol/water partition coefficients at different pH values are listed in Table 1. The Potts and Guy relation was formulated on the assumption that permeation happens across epidermis. In the present disclosure, only stratum corneum was considered for diffusion of fentanyl under the influence of electroporation. To account for the change, the partition coefficient $(K_{sc/vehicle})$ was corrected by volume ratio factor.

**Table 1:** Octanol/water partition coefficients at different pH values

| pH | 8.4 | 7.4 | 5.73 | 5.0 | 4.0 |
|---|---|---|---|---|---|
| $Ko/w$ | 860 | 816 | 717 | 703.55 | 657.27 |

The remaining parameters were obtained from various sources and are listed in Table 2 as shown below:

**Table 2:** Physical properties (referred as properties) used in the FEM simulations

| Physical property | Value | Source |
|---|---|---|
| Electrical conductivity ($\sigma$) | 0.53 S/m | Hulcova et al. (Modelling and validation of dielectric properties of human skin in the MHz region focusing on skin layer morphology and material composition. Journal of Physics D: Applied Physics, 45(2), p.025301.) |
| Electrophoretic mobility ($m$) | 2.85 x 10$^{-8}$ m$^2$/V.s | Einstein-Smoluchowski relation |

(continued)

| Physical property | Value | Source |
|---|---|---|
| Passive diffusion coefficient ($D_{passive}$) | $3.67 \times 10^{-10}$ m$^2$/s | Gajula et al. (In-Silico skin model: a multiscale simulation study of drug transport. Journal of chemical information and modeling, 57(8), pp.2027-2034.) |
| Active diffusion coefficient ($D_{active}$) | $7.67 \times 10^{-10}$ m$^2$/s | Present disclosure |
| Partition coefficient ($K_{sc/vehicle}$) | 100.14 | Potts and Guy relation (1992. Predicting skin permeability. Pharmaceutical research, 9(5), pp.663-669.) |

The simulation parameters were identical to that of electroporation experiment. Initially at t=0, 15 exponentially decaying electric pulses, having 1 pulse/minute rate and 200ms duration, were employed. The applied voltage during electroporation was 500 V which resulted in a transdermal voltage of 46 ~ 70 V. The total time of studying the transport 28800s (8h) was divided into electroporation time of 900s (15 exponentially decay pulses) and passive transport of 27900s (7.75h). The Fentanyl concentration of 5 mg/ml in donor solution was used. The charge state of fentanyl in the all the simulations was +1.

The value of diffusion coefficient obtained during electroporation from MD simulations was approximately 2 times that of passive case. The literature experiment showed an increase in flux of fentanyl through skin epidermis by 270 times during electroporation in comparison to passive diffusion case. The increased flux through skin SC could not be obtained using MD simulations alone due to associated length and time scales. To understand the detailed mechanism, two cases were studied/experimented by the present disclosure: 1) Macroscopic passive diffusion model with an active and passive diffusion coefficient, and 2) Macroscopic electroporation model (solving Nernst-Planck equation and Laplacian equation for voltage). Flux through SC layer was calculated from the concentration gradient. The cumulative release of fentanyl through SC layer was obtained by time integration of flux. In the design of experiment study, five square wave pulses, at the rate of 1 pulse/minute, with pulse duration varied from t1 = 0.133s, t2 = 0.219s and t3 = 0.327s, were used. The applied voltage varied from 50V, 100V, 150V and 250V. The respective transdermal voltages were 20V, 30V, 50V and 60V. The total time of studying the transport 21600s (6h) was divided into electroporation time of 300s (five square wave pulses) and passive transport of 21300s. Fentanyl concentration of 40 ug/ml in donor solution was used. A full factorial design simulation was performed and flux and cumulative releases were calculated.

The macroscopic passive diffusion model was taken from conventional research work (e.g., refer India patent application: IN201721007631 titled "METHOD AND SYSTEM FOR IN-SILICO TESTING OF ACTIVES ON HUMAN SKIN" Filed on March 3, 2017). This model was used to calculate the flux and cumulative release of drug molecular under passive diffusion case. Simulations were performed to check whether the thickness of SC layer is affecting the amount of fentanyl permeated through it. Flux and cumulative release decreased with increase in thickness of SC layer, which were in line with the experimental observations. In the subsequent simulations, SC thickness of 21.5 $\mu$m was considered to study the effect of electroporation fentanyl transport.

**Macroscopic passive diffusion model with an active diffusion coefficient**

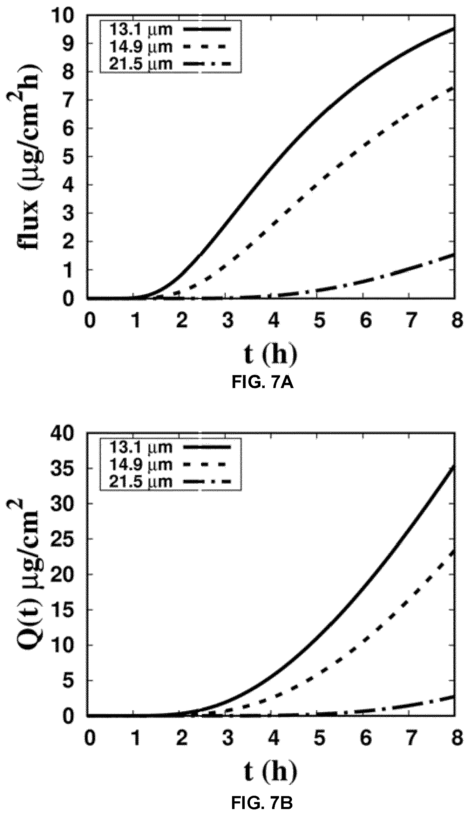

During the electroporation, the pores were formed inside the skin SC lipid layer as shown in FIG. 4. The fentanyl drug crossed the skin lipid layer within few nanoseconds through the created pore due to application of external electric field (FIGS. 5-6). The diffusion of fentanyl during electroporation (active case) increased almost 2 times as compared to passive mode. To understand whether only two times increment of diffusion can explain the multifold increase in the flux during the electroporation, the fentanyl transport across the macroscopic model was used with an active diffusion coefficient. In the first case, passive diffusion model was simulated with $D_{passive}$ alone as input. FIG. 7A, with reference to FIGS. 1 through 6, depicts a graphical representation of change in flux of fentanyl for different thickness of SC layer during passive diffusion, in accordance with an example embodiment of the present disclosure. In the second case, passive diffusion model was simulated with multiple diffusion coefficients ($D_{active}$ and $D_{passive}$). During first 900s (0.25h) $D_{active}$ was used as input to the model and $D_{passive}$ was used from 0.25h to 8h (duration of 27900s). FIG. 7B, with reference to FIGS. 1 through 7A, depicts a graphical representation of change in cumulative release of fentanyl for different thickness of SC layer during passive diffusion, in accordance with an example embodiment of the present disclosure. Both flux and cumulative release decreased with increase in thickness of SC.

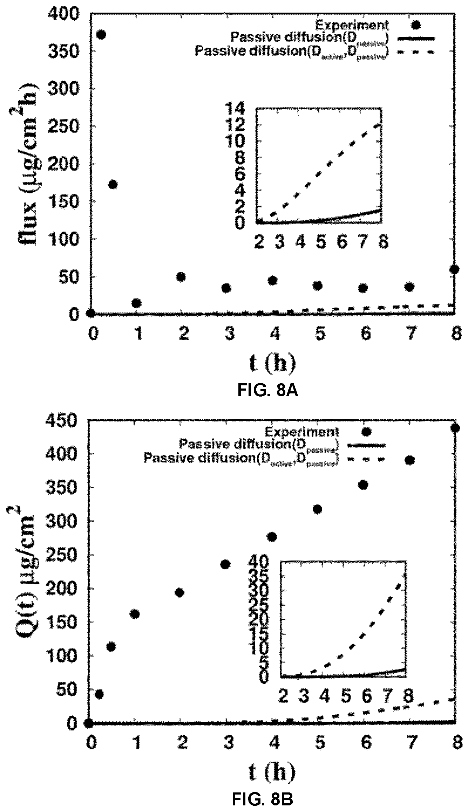

FIG. 8A, with reference to FIGS. 1 through 7B, depicts a graphical representation illustrating a comparison of experimental measured fentanyl flux across skin during electroporation versus flux calculated using a previous multiscale model using two different diffusion coefficients from MD simulations, in accordance with an embodiment of the present disclosure. FIG. 8B, with reference to FIGS. 1 through 8A, depicts a graphical representation illustrating a comparison of experimental measured cumulative release profile of fentanyl flux across skin during electroporation versus cumulative release profiles of fentanyl calculated using the previous multiscale model using two different diffusion coefficients from MD simulations, in accordance with an embodiment of the present disclosure.

More specifically, FIG. 8A shows the rise in flux of fentanyl only after few hours with an active diffusion coefficient. Experiments indicated a sudden rise of flux within few seconds during electroporation, which could not be captured by the passive diffusion model alone. The inclusion of active diffusion coefficient was only able to increase the flux value but not matching with the experiments. FIG. 8B shows the cumulative release of fentanyl at the end of 8h was 2.72 $\mu$g/cm2 and 35.88 $\mu$g/cm2 for first and second case respectively. The quick onset of flux (shorter lag time) during electroporation also could not be determined by only solving diffusion equation with active diffusion coefficient. This confirms that diffusion alone is not able to predict the overall transport of fentanyl through skin during electroporation. Passive diffusion due to lack of electrophoretic effect could not predict desired transport even with inclusion of diffusion coefficient obtained from electroporation MD simulations. In other words, during electroporation, diffusion alone was not able to predict the transport through skin. $D_{passive}$ is the diffusion coefficient of fentanyl through skin SC in passive diffusion conditions and $D_{active}$ is the diffusion coefficient of fentanyl during electroporation. In further simulations, electrophoretic effect along with diffusion is considered to study transport of fentanyl through skin SC during electroporation.

**Macroscopic electroporation model:**

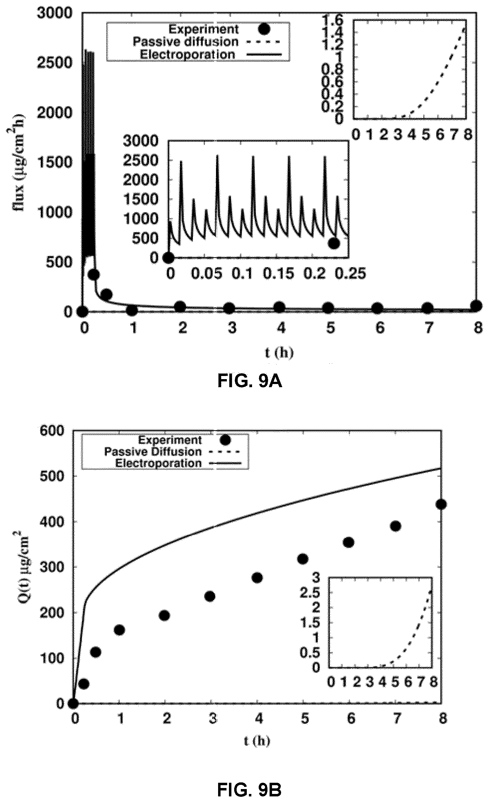

As discussed above, that only increment in diffusion coefficient cannot explain the multi-fold increase in flux. In this case, the passive model was modified to incorporate the electric field effect. The Nernst-Planck equation and Laplacian equations were solved to obtain electric field and concentration profiles across SC layer. The active diffusion coefficient $D_{active}$ was used during electroporation time (15 exponential decay pulses) and passive diffusion coefficient $D_{passive}$ was used during passive diffusion time (after electric field is withdrawn). The calculated flux and cumulative release profiles of fentanyl were plotted in FIGS. 9A and 9B respectively. More specifically, FIG. 9A, with reference to FIGS. 1 through 8B, depicts a graphical representation illustrating a comparison of experimental measured fentanyl flux across skin during electroporation versus calculated fentanyl flux using multiscale model during passive conditions and during electroporation, in accordance with an example embodiment of the present disclosure. FIG. 9B, with reference to FIGS. 1 through 9A, depicts a graphical representation illustrating a comparison of experimental measured cumulative release profile of fentanyl flux across skin during electroporation versus calculated cumulative release using multiscale model during passive conditions and during electroporation, in accordance with an example embodiment of the present disclosure. In other words, FIGS. 9A and 9B show flux and cumulative release profiles of fentanyl upon application of exponential decay voltage pulse during electroporation. At start of each pulse, flux reached a peak value and decayed exponentially through pulse duration (electric field is withdrawn at the end).

From FIG. 9A, intended pulse effect on flux of fentanyl during the electroporation time (0.25h) was obtained. A total of 15 peaks in flux profile (one for each voltage pulse applied) were obtained. At the start of each pulse, the flux rises to a peak value and drops down exponentially towards its end. The peak values at each pulse are different due to the variation in transdermal voltage across SC. The fentanyl flux during electroporation time at each pulse was obtained as a continuous curve wherein experiment reports flux at discrete intervals. Once the electric field was withdrawn, the flux of fentanyl continued to decrease. One of the research works Gupta et al. (Electroporation of Skin Stratum Corneum Lipid Bilayer and Molecular Mechanism of Drug Transport: A Molecular Dynamic Study. Langmuir 2018, 34, 5860-5870), reported the bilayer reformation with resealing of pores in SC lipid bilayer once the electric field is withdrawn. In simulations performed by the present disclosure, systems and methods could obtain this effect with the decrement of flux across SC (after electric field is withdrawn) indicating closure of pores in SC lipid bilayer.

As mentioned above, FIG. 9B shows the comparison between the cumulative release profiles obtained from multiscale model of skin electroporation with the experiments. The cumulative amount of fentanyl at the end of 8hrs were 517.28 $\mu$g/cm2 and 438 $\mu$g/cm2 for macroscopic electroporation model and experiments respectively. In the passive diffusion case at the end of 8hrs, the cumulative amount of fentanyl crossed through SC layer was very negligible compared to experiment. The steady state flux and lag time were obtained for above-mentioned cases by calculating slope of linear part of cumulative release curve and its intercept respectively.

The steady state flux obtained from macroscopic electroporation model was 21.61 $\mu$g/cm$^2$h and it was comparable to experimentally reported value of 22.43 $\mu$g/cm$^2$h. The passive diffusion model showed a lag time of 17.24h to reach the steady state. In the experiments, the lag time was negligible. In the macroscopic electroporation model, also the obtained lag time was negligible. The magnitude of cumulative release, steady state flux and lag time obtained from multiscale model were comparable to that of experiments, the difference between obtained cumulative release profile from simulation and experiments could be because of the thickness of SC layer being considered. In the previous experiments, full thickness of human epidermis was used. Whereas in the simulations performed by the present disclosure only SC layer was considered. The thicknesses of epidermis and SC are reported to be $51.2 \pm 12.2$ $\mu$m and $18.2 \pm 3.3$ $\mu$m respectively. Fentanyl molecule must cross the entire thickness of epidermis in the previously conducted experiments that results in lesser cumulative release. Whereas in simulations performed by the present disclosure, it has to cross only SC layer that results in higher cumulative release. The difference in obtained cumulative release profile compared to experiments could also be due to partition coefficient between vehicle and SC used. The partition coefficient was calculated from correlation with extrapolated water/octanol partition coefficient. Vanbever et al. (Transdermal delivery of metoprolol by electroporation. Pharm. Res. 1994, 11, 1657-1662) reported that the overall transport of molecule is not only due to creation of dynamic aqueous pores but also due to electrophoretic drift. In multiscale model of the present disclosure, this effect was obtained by integrating simulations at both molecular and macroscopic level.

**Design of skin electroporation experiments**

The electroporation protocol consists of setting up electric pulse with various pulse parameters such as voltage, pulse shape (or pulse type), pulse duration and number of pulses per unit time to name few. These parameters are required to be tuned for a specific application to get desired release. The applied voltage has direct effect of electroporation time as shown in FIG. 4. Further, present disclosure also shows, how one can design electroporation experiments, in-silico, using framework presented in earlier paragraphs of the present disclosure.

In this design of experiment study, five square wave pulses, at the rate of 1 pulse/minute, with pulse duration varied from t1 = 0.133s, t2 = 0.219s and t3 = 0.327s, were used. The simulation parameters were identical to the experimental conditions. The applied voltage varied from 50V, 100V, 150V and 250V. The respective transdermal voltages were 20V, 30V, 50V and 60V. The total time of studying the transport 21600s (6h) was divided into electroporation time of 300s (five square wave pulses) and passive transport of 21300s. The Fentanyl concentration of 40 ug/ml in donor solution was used. A full factorial design simulation was performed, and flux and cumulative releases were calculated.

Both voltage and pulse duration act as crucial parameters in obtaining flux. Flux increased upon the application of each pulse and reached to a peak value each time during the pulse duration. The peak value of flux increased during each subsequent pulse and reached to a constant value. For lower pulse duration and lower voltage, flux during initial pulse remained lower compared to that during subsequent pulses by several orders of magnitude. At higher voltages and higher pulse durations the initial peak appeared was larger

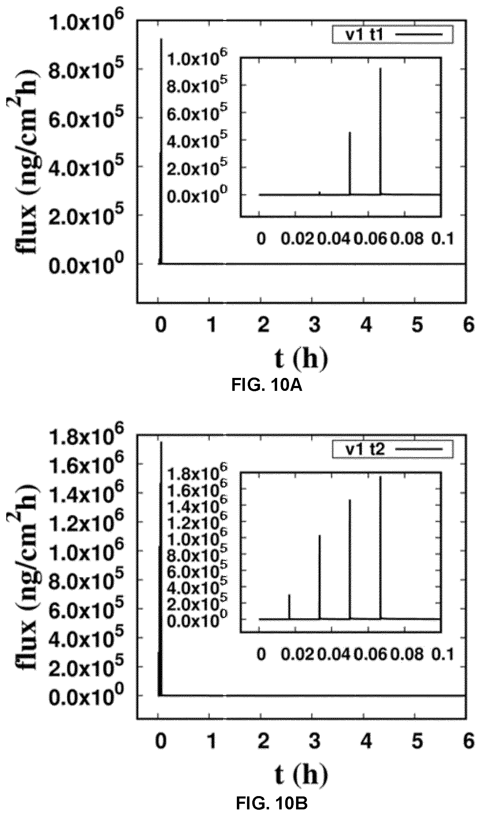

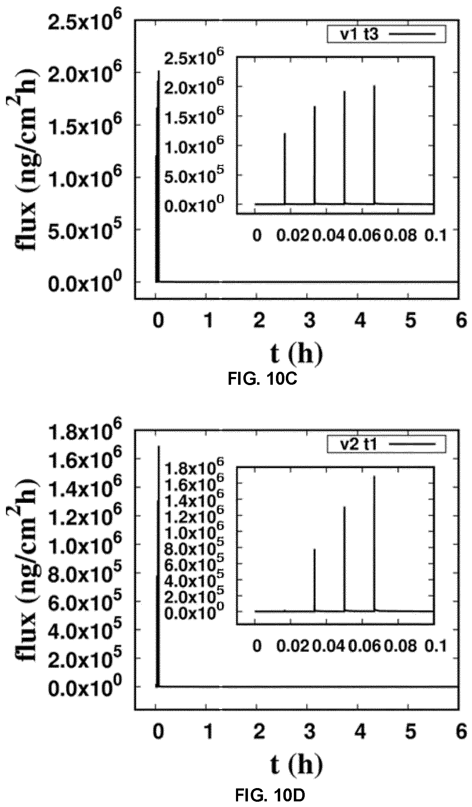

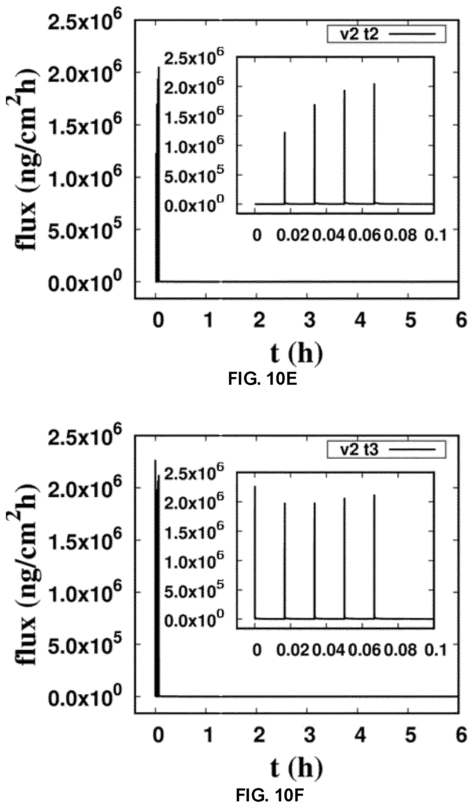

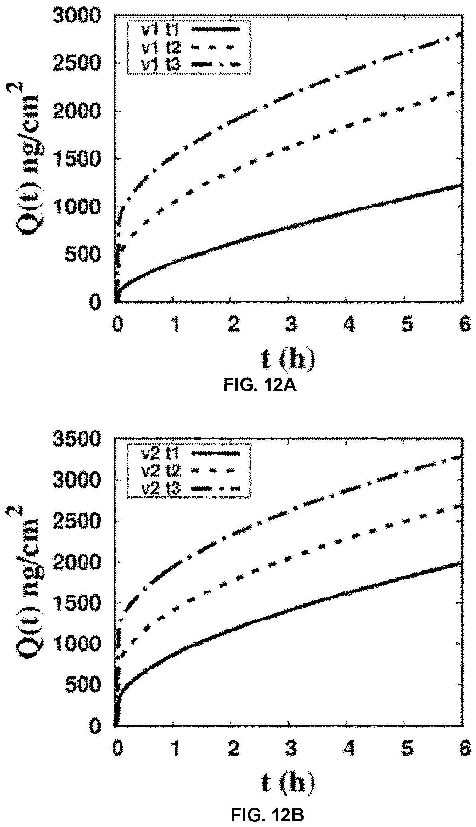

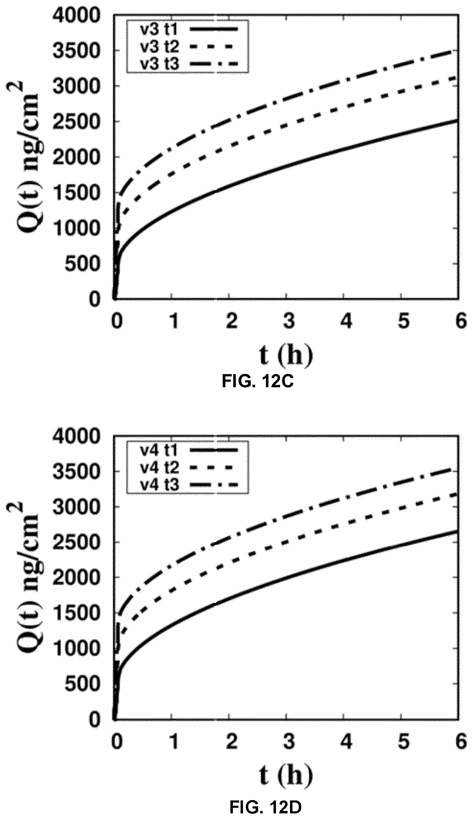

For a given voltage applied, flux increased with increase in pulse duration, which allowed more amount of fentanyl to permeate through. For given pulse duration, flux increased with increase in voltage applied allowing fentanyl to experience greater electrophoretic effect. FIGS. 10A through 11F, with reference to FIGS. 1 through 9B, depict fentanyl flux profiles for factorial simulations, in accordance with an example embodiment of the present disclosure. At each pulse, flux reached to a peak value and continued to decrease once the electric field is withdrawn. At lower voltages and lower pulse durations, the initial flux peak remained low compared to subsequent peaks. At these conditions pore sizes are not big enough for permeation of fentanyl molecules. The peak value of flux increased with increase in pulse duration for a given voltage. The cumulative release profiles were obtained by time integrating the flux profiles and are plotted and depicted in FIGS. 12A-12D. More specifically, FIG. 12A through 12D, with reference to FIGS. 1 through 11F, depicts cumulative release profiles for factorial simulations, in accordance with an example embodiment of the present disclosure. The factorial simulations consist of four voltages and three pulse durations, in an example embodiment. More specifically, FIG. 12 depicts cumulative release of fentanyl ($ng/cm^2$) at different voltages and pulse durations from factorial design simulations. Voltages of v1 (50V), v2 (100V), v3 (150V) and v4 (250V) were considered. Pulse durations of t1 (0.133s), t2 (0.219s) and t3 (0.327s) were considered. Plots (a), (b), (c) and (d) corresponds to v1, v2, v3 and v4 respectively. Cumulative release increased with increase in pulse duration for a given voltage. Longer duration of pulse allowed pore at molecular level to grow bigger thereby increased amount of fentanyl permeated through.

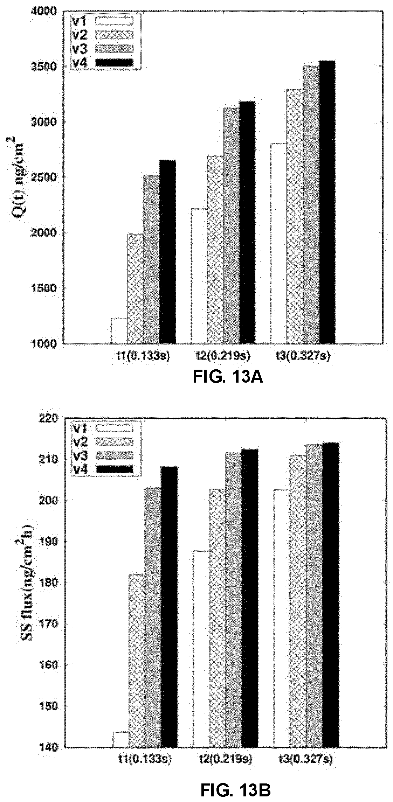

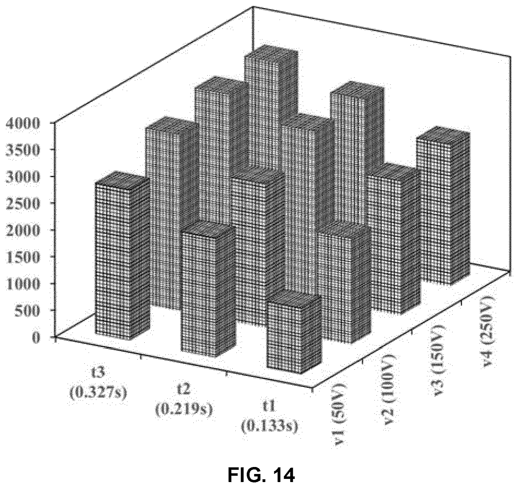

FIGS. 13A-13B, with reference to FIGS. 1 through 12D, depict a comparative study of cumulative release at 6hrs and steady state flux for different voltages and different pulse durations, in accordance with an example embodiment of the present disclosure. More specifically, in FIG. 13A, the representation depicts cumulative release of fentanyl ($ng/cm^2$) at the end of 6hrs for different voltages and different pulse durations and in FIG. 13B, the representation depicts steady state flux ($ng/cm^2h$) for different voltages and different pulse durations. Different applied voltages were v1 (50V), v2 (100V), v3 (150V) and v4 (250V). Different pulse durations were t1 (0.133s), t2 (0.219s) and t3 (0.327s). Cumulative amount of fentanyl permeated through skin at end of 6hrs increased as voltage was increased and approached to a constant value indicating that bigger size of pore thereby allowing maximum number of fentanyl molecules through it. It was also evident from the steady state flux calculations, which approached to a constant value as voltage increased. Cumulative release increased as pulse duration increased for a given applied voltage. It indicated that, as longer pulse duration allowed pore to grow bigger to facilitate large number of fentanyl molecules to pass through. The same was obtained using steady state flux calculations showing higher was steady state flux for longer pulse duration. The surface plot consists of factorial number of design simulations were plotted in FIG. 14. More specifically, FIG. 14, with reference to FIGS. 1 through 13B, depicts a design space for selecting an electroporation protocol for specific application, in accordance with an example embodiment of the present disclosure. More specifically, FIG. 14 depicts cumulative release of fentanyl ($ng/cm^2$) at the end of 6hrs for factorial design simulations. Voltages of v1 (50V), v2 (100V), v3 (150V) and v4 (250V) were considered. Pulse durations of t1 (0.133s), t2 (0.219s) and t3 (0.327s) were considered. In FIG. 14, variation of cumulative release profile with respect to voltage and pulse duration for fixed pulse rate and pulse shape is observed. The parameters of electroporation protocol can be tuned for a specific application. In other words, the systems and methods of the present disclosure enable the hardware processors 104 to fine-tune the one or more pre-defined pulse parameters based on the calculated flux and cumulative release profile.

Systems and methods of the present disclosure provide various embodiments that implement a multiscale model for electroporation by integrating molecular model and macroscopic models. In the present disclosure, systems and methods studied effect of SC thickness on transport and it was observed that increase in thickness of SC results in lower permeation of drug. Further, the effect of computed diffusion coefficients, as obtained from MD simulations, on the transport of drug was also investigated. During investigation, it was observed that using molecular level information alone is not sufficient to predict the transport of drug through SC. Finally, the influence of electroporation on release profile of fentanyl through SC under experimental conditions was investigated. Accurately obtained diffusion coefficients ($D_{passive}$ and $D_{active}$) from molecular dynamics simulations were incorporated into the multiscale model. In the macroscopic model, Laplacian and Nernst-Planck equations were solved. Fentanyl flux and cumulative release through human skin SC were calculated and found to be comparable with the experiments. The developed multiscale model can be used to plan specific electroporation protocols for studying active transport through skin. The design case was illustrated with an example of delivering fentanyl through skin for different voltages and pulse durations, and such example of fentanyl shall not be construed as limiting the scope of the present disclosure and its embodiments described herein.

Conventional systems and methods limit their approaches for simpler stratum corneum (SC) model and approximated parameters and thus lacked in understanding of electroporation. Further, these approaches limit their capabilities to a specific phenomenon either molecular of macroscopic level permeation, and flux and cumulative release profiles could not be obtained using molecular models due to their limitations with respect to time and length scales. Conventional simulation studies also lack in designing electroporation experimental protocols which require selection of multiple pulse parameters. Embodiments of the present disclosure provide systems and methods for in-silico design of skin electroporation parameters; wherein skin lipids membrane are simulated for calculation of physical properties such as diffusion coefficient. Further, macroscopic diffusion is simulated during electroporation and flux and cumulative release profiles of actives are computed. The system of the present disclosure may be utilized as a design tool for selecting suitable electroporation protocol from factorial simulations. In other words, unlike conventional systems/methods and research works, embodiments of the present disclosure present a molecular level resolution and model of realistic skin lipid matrix, wherein *in-silico* design of skin electroporation experiments is provided for better results. The system acts as a tool for virtual testing of active permeation through skin in presence of external electric field. Further, unlike conventional approaches wherein there was no integration of macroscopic models with molecular level understanding of electroporation, embodiments of the present disclosure implement an integration of molecular and macroscopic level phenomena, thus helping in designing better electroporation protocol.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with the scope of the invention being indicated by the following claims.

**Claims**

1. A processor implemented method, comprising:

   obtaining (202), via one or more hardware processors, a molecular electroporation model of a lipid bilayer of a stratum corneum layer of a human skin;
   applying (204), via the one or more hardware processors, a proration electric field across the lipid bilayer which forms a pore inside the lipid bilayer of the stratum corneum layer;
   stabilizing (206), via the one or more hardware processors, the pore by reducing the applied electric field and obtaining a stable pore;
   introducing (208), via the one or more hardware processors, one or more drug molecules on the lipid bilayer having the stable pore in the presence of a stable electric field;
   calculating (210), using a molecular dynamics, MD, simulation technique executed by the one or more hardware processors, an active diffusion coefficient and a passive diffusion coefficient of the one or more drug molecules through the stable pore, wherein the active diffusion coefficient refers to a diffusion coefficient obtained in presence of electric field and passive diffusion coefficient refers to a diffusion coefficient obtained in absence of electric field;
   generating (212), via the one or more hardware processors, a skin macroscopic structure of the stratum corneum layer of the human skin, wherein the stratum corneum layer of the human skin comprises corneocytes embedded in the form of a lipid matrix;
   calculating (214), using the diffusion coefficients obtained using the molecular electroporation model of the lipid bilayer of the stratum corneum of the human skin, via a finite element analysis, FEA, technique executed by the one or more hardware processors, a concentration gradient of the one or more drug molecules in the presence of the applied proration electric field, wherein calculating the concentration gradient comprises solving a Laplacian equation and Nernst-Planck equation in lipid regions of the stratum corneum layer respectively, based on -i-one or more pre-defined boundary conditions of the macroscopic structure of the stratum corneum layer, -ii-one or more pre-defined pulse parameters and -iii-one or more properties obtained from the molecular electroporation model,

      wherein the Laplacian equation is given by: $\nabla.\,(\sigma\nabla V) = 0$,
      wherein the Nernst-Planck equation is given by:

$$\frac{\partial C}{\partial t} = \nabla.\,[(D\nabla C) + (mC\nabla V)],$$

      where, $C$ = concentration of permeating drug molecule, $D$ = active/passive diffusion coefficient of drug molecule in stratum corneum lipid region, $V$ = voltage applied across the human skin, $\sigma$ = electrical conductivity and $m$ = electrophoretic mobility, and
      wherein the electrophoretic mobility is calculated using an Einstein-Smoluchowski relation given by:

$$m = \frac{z*D*F}{R*T}$$

where, $D$ is the active/passive diffusion coefficient of the drug molecule in stratum corneum, $z$ is charge of the drug molecule, $F$ is faraday's constant, $R$ is gas constant and $T$ is temperature; and

calculating (216), via the one or more hardware processors, a flux profile and a cumulative release profile of the one or more drug molecules using the concentration gradient obtained using the FEA technique;
adjusting the one or more pre-defined pulse parameters based on the calculated flux profile and the calculated cumulative release profile, by using a full factorial design simulation to select an electroporation protocol to get desired release profile for the one or more drug molecules, wherein the electroporation protocol consists of adjusting the one or more pre-defined pulse parameters to get the desired release profile for the one or more drug molecules.

2. The processor implemented method as claimed in claim 1, wherein the one or more pre-defined pulse parameters comprise at least one of a pulse duration, a pulse type, and an applied voltage.

3. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

obtain a molecular electroporation model of a lipid bilayer of a stratum corneum layer of a human skin;
apply an electric field across the lipid bilayer which forms a pore inside the lipid bilayer of the stratum corneum layer;
stabilize the pore to obtain a stable pore by reducing the applied electric field and obtain a stable pore;
introduce one or more drug molecules on the lipid bilayer having the stable pore in the presence of the electric field;
calculate, using a molecular dynamics, MD, simulation technique, an active diffusion coefficient and a passive diffusion coefficient of the one or more drug molecules through the stable pore, wherein the active diffusion coefficient refers to a diffusion coefficient obtained in presence of electric field and passive diffusion coefficient refers to a diffusion coefficient obtained in absence of electric field;
generate a skin macroscopic structure of the stratum corneum layer of the human skin, wherein the stratum corneum layer of the human skin comprises corneocytes embedded in the form of a lipid matrix; calculate, using the diffusion coefficients obtained using the molecular electroporation model of the lipid bilayer of the stratum corneum of the human skin, via a finite element analysis technique, FEA,
a concentration gradient of the one or more drug molecules in the presence of the electric field, wherein the concentration gradient is calculated by solving a Laplacian equation and Nernst-Planck equation in lipid regions of the stratum corneum layer respectively, based on -i-one or more pre-defined boundary conditions of the macroscopic structure of the stratum corneum layer, -ii-one or more pre-defined pulse parameters and -iii-one or more properties obtained from the molecular electroporation model,
wherein the Laplacian equation is given by: $\nabla . (\sigma \nabla V) = 0$,
wherein the Nernst-Planck equation is given by:

$$\frac{\partial C}{\partial t} = \nabla . [(D\nabla C) + (mC\nabla V)],$$

where, $C$ = concentration of permeating drug molecule, $D$ = active/passive diffusion coefficient of drug molecule in stratum corneum lipid region, $V$ = voltage applied across the human skin, $\sigma$ = electrical conductivity and $m$ = electrophoretic mobility , and
wherein the electrophoretic mobility is calculated using an Einstein-Smoluchowski relation given by:

$$m = \frac{z*D*F}{R*T}$$

where, $D$ is the active/passive diffusion coefficient of the drug molecule in stratum corneum, $z$ is charge of the drug molecule, $F$ is faraday's constant, $R$ is gas constant and $T$ is temperature; and
calculate a flux profile and a cumulative release profile of the one or more drug molecules using the concentration gradient obtained using the FEA technique;
adjusting the one or more pre-defined pulse parameters based on the calculated flux profile and the calculated cumulative release profile, by using a full factorial design simulation to select an electroporation protocol to get desired release profile for the one or more drug molecules, wherein the electroporation protocol consists of adjusting the one or more pre-defined pulse parameters to get the desired release profile for the one or more drug molecules.

4. The system (100) as claimed in claim 3, wherein the one or more pre-defined pulse parameters comprise at least one of a pulse duration, a pulse type, and an applied voltage.

5. One or more non-transitory machine-readable information storage mediums comprising one or more instructions which when executed by one or more hardware processors cause in-silico design of electroporation experiments for topical and transdermal drug delivery applications by:

obtaining, via the one or more hardware processors, a molecular electroporation model of a lipid bilayer of a stratum corneum layer of a human skin;

applying, via the one or more hardware processors, an electric field across the lipid bilayer which forms a pore inside the lipid bilayer of the stratum corneum layer;

stabilizing, via the one or more hardware processors, the pore by reducing the applied electric field to obtain a stable pore;

introducing, via the one or more hardware processors, one or more drug molecules in the stable pore in the presence of the electric field;

calculating, using a molecular dynamics, MD, simulation technique executed by the one or more hardware processors, an active diffusion coefficient and a passive diffusion coefficient of the one or more drug molecules on the lipid bilayer having the stable pore, wherein the active diffusion coefficient refers to a diffusion coefficient obtained in presence of electric field and passive diffusion coefficient refers to a diffusion coefficient obtained in absence of electric field;

generating, via the one or more hardware processors, a skin macroscopic structure of the stratum corneum layer of the human skin, wherein the stratum corneum layer of the human skin comprises corneocytes embedded in the form of a lipid matrix;

calculating, using the diffusion coefficients obtained using the molecular electroporation model of the lipid bilayer of the stratum corneum of the human skin, via a finite element analysis technique executed by the one or more hardware processors, a concentration gradient of the one or more drug molecules in the presence of the applied electric field, wherein calculating the concentration gradient comprises solving a Laplacian equation and Nernst-Planck equation in lipid regions of the stratum corneum layer respectively, based on -i-one or more pre-defined boundary conditions of the macroscopic structure of the stratum corneum layer, -ii-one or more pre-defined pulse parameters and -iii-one or more properties obtained from the molecular electroporation model,

wherein the Laplacian equation is given by: $\nabla.\,(\sigma\nabla V) = 0,$
wherein the Nernst-Planck equation is given by:

$$\frac{\partial C}{\partial t} = \nabla.\left[(D\nabla C) + (mC\nabla V)\right],$$

where, $C$ = concentration of permeating drug molecule, $D$ = active/passive diffusion coefficient of drug molecule in stratum corneum lipid region, $V$ = voltage applied across the human skin, $\sigma$ = electrical conductivity and $m$ = electrophoretic mobility , and
wherein the electrophoretic mobility is calculated using an Einstein-Smoluchowski relation given by:

$$m = \frac{z * D * F}{R * T}$$

where, $D$ is the active/passive diffusion coefficient of the drug molecule in stratum corneum, $z$ is charge of the drug molecule, $F$ is faraday's constant, $R$ is gas constant and $T$ is temperature; and

calculating, via the one or more hardware processors, a flux profile and a cumulative release profile of the one or more drug molecules using the concentration gradient obtained using the finite element analysis technique;

adjusting the one or more pre-defined pulse parameters based on the calculated flux profile and the calculated cumulative release profile, by using a full factorial design simulation to select an electroporation protocol to get desired release profile for the one or more drug molecules, wherein the electroporation protocol consists of adjusting the one or more pre-defined pulse parameters to get the desired release profile for the one or more drug molecules.

**6.** The one or more non-transitory machine-readable information storage mediums of claim 5, wherein the one or more pre-defined pulse parameters comprise at least one of a pulse duration, a pulse type, and an applied voltage.

**Patentansprüche**

**1.** Prozessorimplementiertes Verfahren, umfassend:

Erhalten (202), über einen oder mehrere Hardwareprozessoren, eines molekularen Elektroporationsmodells einer Lipiddoppelschicht einer Stratum corneum-Schicht einer menschlichen Haut;
Anlegen (204), über den einen oder die mehreren Hardwareprozessoren, eines elektrischen Polarisationsfelds über die Lipiddoppelschicht, das eine Pore innerhalb der Lipiddoppelschicht der Stratum corneum-Schicht bildet;
Stabilisieren (206), über den einen oder die mehreren Hardwareprozessoren, der Pore durch Reduzieren des angelegten elektrischen Felds und Erhalten einer stabilen Pore;
Einführen (208), über den einen oder die mehreren Hardwareprozessoren, eines oder mehrerer Arzneimittelmoleküle auf die Lipiddoppelschicht mit der stabilen Pore in Gegenwart eines stabilen elektrischen Felds;
Berechnen (210), unter Verwendung einer Molekulardynamik-, MD-, Simulationstechnik, die von dem einen oder den mehreren Hardwareprozessoren ausgeführt wird, eines aktiven Diffusionskoeffizienten und eines passiven Diffusionskoeffizienten des einen oder der mehreren Arzneimittelmoleküle durch die stabile Pore, wobei sich der aktive Diffusionskoeffizient auf einen Diffusionskoeffizienten bezieht, der in Gegenwart eines elektrischen Felds erhalten wird, und sich der passive Diffusionskoeffizient auf einen Diffusionskoeffizienten bezieht, der in Abwesenheit eines elektrischen Felds erhalten wird;
Erzeugen (212), über den einen oder die mehreren Hardwareprozessoren, einer makroskopischen Hautstruktur der Stratum corneum-Schicht der menschlichen Haut, wobei die Stratum corneum-Schicht der menschlichen Haut Corneocyten umfasst, die in Form einer Lipidmatrix eingebettet sind;
Berechnen (214), unter Verwendung der Diffusionskoeffizienten, die unter Verwendung des molekularen Elektroporationsmodells der Lipiddoppelschicht der Stratum corneum der menschlichen Haut erhalten werden, über eine Finite-Elemente-Analyse-, FEA-, Technik, die von dem einen oder den mehreren Hardwareprozessoren ausgeführt wird, eines Konzentrationsgradienten des einen oder der mehreren Arzneimittelmoleküle in Gegenwart des angelegten elektrischen Polarisationsfelds, wobei das Berechnen des Konzentrationsgradienten das Lösen einer Laplace-Gleichung und einer Nernst-Planck-Gleichung in Lipidregionen der Stratum corneum-Schicht jeweils basierend auf -i- einer oder mehreren vordefinierten Randbedingungen der makroskopischen Struktur der Stratum corneum-Schicht, -ii- einem oder mehreren vordefinierten Impulsparametern und -iii- einer oder mehreren Eigenschaften, die aus dem molekularen Elektroporationsmodell erhalten werden, umfasst,

wobei die Laplace-Gleichung gegeben ist durch: $\nabla \cdot (\sigma \nabla V) = 0$,
wobei die Nernst-Planck-Gleichung gegeben ist durch:

$$\frac{\partial C}{\partial t} = \nabla \cdot [(D\nabla C) + (mC\nabla V)],$$

wobei $C$ = Konzentration des permeierenden Arzneimittelmoleküls, $D$ = aktiver/passiver Diffusionskoeffizient des Arzneimittelmoleküls in der Stratum corneum-Lipidregion, $V$ = an die menschliche Haut angelegte Spannung, $\sigma$ = elektrische Leitfähigkeit und $m$ = elektrophoretische Mobilität, und
wobei die elektrophoretische Mobilität unter Verwendung einer Einstein-Smoluchowski-Beziehung berechnet wird, die gegeben ist durch: $m = \frac{z*D*F}{R*T}$

wobei $D$ der aktive/passive Diffusionskoeffizient des Arzneimittelmoleküls in Stratum corneum ist, $z$ die Ladung des Arzneimittelmoleküls ist, $F$ die Faraday-Konstante ist, $R$ die Gaskonstante ist und $T$ die Temperatur ist; und

Berechnen (216), über den einen oder die mehreren Hardwareprozessoren, eines Flussprofils und eines kumulativen Freisetzungsprofils des einen oder der mehreren Arzneimittelmoleküle unter Verwendung des Konzentrationsgradienten, der unter Verwendung der FEA-Technik erhalten wird;
Anpassen des einen oder der mehreren vordefinierten Impulsparameter basierend auf dem berechneten Flussprofil und dem berechneten kumulativen Freisetzungsprofil unter Verwendung einer vollfaktoriellen Designsimulation, um ein Elektroporationsprotokoll auszuwählen, um ein gewünschtes Freisetzungsprofil für das eine oder die mehreren Arzneimittelmoleküle zu erhalten, wobei das Elektroporationsprotokoll aus dem Anpassen des einen oder der mehreren vordefinierten Impulsparameter besteht, um das gewünschte Freisetzungsprofil für das eine oder die mehreren Arzneimittelmoleküle zu erhalten.

2. Prozessorimplementiertes Verfahren nach Anspruch 1, wobei der eine oder die mehreren vordefinierten Impulsparameter mindestens eines von einer Impulsdauer, einem Impulstyp und einer angelegten Spannung umfassen.

3. System (100), umfassend:

einen Speicher (102), der Anweisungen speichert;
eine oder mehrere Kommunikationsschnittstellen (106); und
einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:

Erhalten eines molekularen Elektroporationsmodells einer Lipiddoppelschicht einer Stratum corneum-Schicht einer menschlichen Haut;
Anlegen eines elektrischen Felds über die Lipiddoppelschicht, das eine Pore innerhalb der Lipiddoppelschicht der Stratum corneum-Schicht bildet;
Stabilisieren der Pore, um eine stabile Pore zu erhalten, durch Reduzieren des angelegten elektrischen Felds und Erhalten einer stabilen Pore;
Einführen eines oder mehrerer Arzneimittelmoleküle auf die Lipiddoppelschicht mit der stabilen Pore in Gegenwart des elektrischen Felds;
Berechnen, unter Verwendung einer Moleklardynamik-, MD-, Simulationstechnik, eines aktiven Diffusionskoeffizienten und eines passiven Diffusionskoeffizienten des einen oder der mehreren Arzneimittelmoleküle durch die stabile Pore, wobei sich der aktive Diffusionskoeffizient auf einen Diffusionskoeffizienten bezieht, der in Gegenwart eines elektrischen Felds erhalten wird, und sich der passive Diffusionskoeffizient auf einen Diffusionskoeffizienten bezieht, der in Abwesenheit eines elektrischen Felds erhalten wird;
Erzeugen einer makroskopischen Hautstruktur der Stratum corneum-Schicht der menschlichen Haut, wobei die Stratum corneum-Schicht der menschlichen Haut Corneocyten umfasst, die in Form einer Lipidmatrix eingebettet sind; Berechnen, unter Verwendung der Diffusionskoeffizienten, die unter Verwendung des molekularen Elektroporationsmodells der Lipiddoppelschicht der Stratum corneum der menschlichen Haut erhalten werden, über eine Finite-Elemente-Analyse-, FEA-, Technik, eines Konzentrationsgradienten des einen oder der mehreren Arzneimittelmoleküle in Gegenwart des elektrischen Felds, wobei der Konzentrationsgradient durch Lösen einer Laplace-Gleichung und einer Nernst-Planck-Gleichung in Lipidregionen der Stratum corneum-Schicht jeweils basierend auf -i- einer oder mehreren vordefinierten Randbedingungen der makroskopischen Struktur der Stratum corneum-Schicht, -ii- einem oder mehreren vordefinierten Impulsparametern und -iii- einer oder mehreren Eigenschaften, die aus dem molekularen Elektroporationsmodell erhalten werden, berechnet wird,
wobei die Laplace-Gleichung gegeben ist durch: $\nabla \cdot (\sigma \nabla V) = 0$,
wobei die Nernst-Planck-Gleichung gegeben ist durch:

$$\frac{\partial C}{\partial t} = \nabla \cdot [(D \nabla C) + (mC\nabla V)],$$

wobei $C$ = Konzentration des permeierenden Arzneimittelmoleküls, $D$ = aktiver/passiver Diffusionskoeffizient des Arzneimittelmoleküls in der Stratum corneum-Lipidregion, $V$ = an die menschliche Haut angelegte Spannung, $\sigma$ = elektrische Leitfähigkeit und $m$ = elektrophoretische Mobilität, und
wobei die elektrophoretische Mobilität unter Verwendung einer Einstein-Smoluchowski-Beziehung berechnet wird, die gegeben ist durch: $m = \dfrac{z * D * F}{R * T}$

wobei $D$ der aktive/passive Diffusionskoeffizient des Arzneimittelmoleküls in Stratum corneum ist, $z$ die Ladung des Arzneimittelmoleküls ist, $F$ die Faraday-Konstante ist, $R$ die Gaskonstante ist und $T$ die Temperatur ist; und
Berechnen eines Flussprofils und eines kumulativen Freisetzungsprofils des einen oder der mehreren Arzneimittelmoleküle unter Verwendung des Konzentrationsgradienten, der unter Verwendung der FEA-Technik erhalten wird;
Anpassen des einen oder der mehreren vordefinierten Impulsparameter basierend auf dem berechneten Flussprofil und dem berechneten kumulativen Freisetzungsprofil unter Verwendung einer vollfaktoriellen Designsimulation, um ein Elektroporationsprotokoll auszuwählen, um ein gewünschtes Freisetzungsprofil für das eine oder die mehreren Arzneimittelmoleküle zu erhalten, wobei das Elektroporationsprotokoll aus dem Anpassen des einen oder der mehreren vordefinierten Impulsparameter besteht, um das gewünschte Freisetzungsprofil für das eine oder die mehreren Arzneimittelmoleküle zu erhalten.

4. System (100) nach Anspruch 3, wobei der eine oder die mehreren vordefinierten Impulsparameter mindestens eines von einer Impulsdauer, einem Impulstyp und einer angelegten Spannung umfassen.

5. Ein oder mehrere nichtflüchtige maschinenlesbare Informationsspeichermedien, die eine oder mehrere Anweisungen umfassen, die, wenn sie von einem oder mehreren Hardwareprozessoren ausgeführt werden, ein In-Silico-Design von Elektroporationsexperimenten für topische und transdermale Arzneimittelabgabeanwendungen bewirken durch:

Erhalten, über den einen oder die mehreren Hardwareprozessoren, eines molekularen Elektroporationsmodells einer Lipiddoppelschicht einer Stratum corneum-Schicht einer menschlichen Haut;
Anlegen, über den einen oder die mehreren Hardwareprozessoren, eines elektrischen Felds über die Lipiddoppelschicht, das eine Pore innerhalb der Lipiddoppelschicht der Stratum corneum-Schicht bildet;
Stabilisieren, über den einen oder die mehreren Hardwareprozessoren, der Pore durch Reduzieren des angelegten elektrischen Felds, um eine stabile Pore zu erhalten;
Einführen, über den einen oder die mehreren Hardwareprozessoren, eines oder mehrerer Arzneimittelmoleküle in die stabile Pore in Gegenwart des elektrischen Felds;
Berechnen, unter Verwendung einer Moleküldynamik-, MD-, Simulationstechnik, die von dem einen oder den mehreren Hardwareprozessoren ausgeführt wird, eines aktiven Diffusionskoeffizienten und eines passiven Diffusionskoeffizienten des einen oder der mehreren Arzneimittelmoleküle auf die Lipiddoppelschicht mit der stabilen Pore, wobei sich der aktive Diffusionskoeffizient auf einen Diffusionskoeffizienten bezieht, der in Gegenwart eines elektrischen Felds erhalten wird, und sich der passive Diffusionskoeffizient auf einen Diffusionskoeffizienten bezieht, der in Abwesenheit eines elektrischen Felds erhalten wird;
Erzeugen, über den einen oder die mehreren Hardwareprozessoren, einer makroskopischen Hautstruktur der Stratum corneum-Schicht der menschlichen Haut, wobei die Stratum corneum-Schicht der menschlichen Haut Corneocyten umfasst, die in Form einer Lipidmatrix eingebettet sind;
Berechnen, unter Verwendung der Diffusionskoeffizienten, die unter Verwendung des molekularen Elektroporationsmodells der Lipiddoppelschicht der Stratum corneum der menschlichen Haut erhalten werden, über eine Finite-Elemente-Analyse-Technik, die von dem einen oder den mehreren Hardwareprozessoren ausgeführt wird, eines Konzentrationsgradienten des einen oder der mehreren Arzneimittelmoleküle in Gegenwart des angelegten elektrischen Felds, wobei das Berechnen des Konzentrationsgradienten das Lösen einer Laplace-Gleichung und einer Nernst-Planck-Gleichung in Lipidregionen der Stratum corneum-Schicht jeweils basierend auf -i- einer oder mehreren vordefinierten Randbedingungen der makroskopischen Struktur der Stratum corneum-Schicht, -ii- einem oder mehreren vordefinierten Impulsparametern und -iii- einer oder mehreren Eigenschaften, die aus dem molekularen Elektroporationsmodell erhalten werden, umfasst,

wobei die Laplace-Gleichung gegeben ist durch: $\nabla \cdot (\sigma \nabla V) = 0$,
wobei die Nernst-Planck-Gleichung gegeben ist durch:

$$\frac{\partial C}{\partial t} = \nabla \cdot [(D\nabla C) + (mC\nabla V)],$$

wobei $C$ = Konzentration des permeierenden Arzneimittelmoleküls, $D$ = aktiver/passiver Diffusionskoeffizient des Arzneimittelmoleküls in der Stratum corneum-Lipidregion, $V$ = an die menschliche Haut angelegte Spannung, $\sigma$ = elektrische Leitfähigkeit und $m$ = elektrophoretische Mobilität, und
wobei die elektrophoretische Mobilität unter Verwendung einer Einstein-Smoluchowski-Beziehung berechnet wird, die gegeben ist durch: $m = \frac{z*D*F}{R*T}$

wobei $D$ der aktive/passive Diffusionskoeffizient des Arzneimittelmoleküls in Stratum corneum ist, $z$ die Ladung des Arzneimittelmoleküls ist, $F$ die Faraday-Konstante ist, $R$ die Gaskonstante ist und $T$ die Temperatur ist; und

Berechnen, über den einen oder die mehreren Hardwareprozessoren, eines Flussprofils und eines kumulativen Freisetzungsprofils des einen oder der mehreren Arzneimittelmoleküle unter Verwendung des Konzentrationsgradienten, der unter Verwendung der Finite-Elemente-Analysetechnik erhalten wird;

Anpassen des einen oder der mehreren vordefinierten Impulsparameter basierend auf dem berechneten Flussprofil und dem berechneten kumulativen Freisetzungsprofil unter Verwendung einer vollfaktoriellen Designsimulation, um ein Elektroporationsprotokoll auszuwählen, um ein gewünschtes Freisetzungsprofil für das eine oder die mehreren Arzneimittelmoleküle zu erhalten, wobei das Elektroporationsprotokoll aus dem Anpassen des einen oder der mehreren vordefinierten Impulsparameter besteht, um das gewünschte Freisetzungsprofil für das eine oder die mehreren Arzneimittelmoleküle zu erhalten.

**6.** Ein oder mehrere nichtflüchtige maschinenlesbare Informationsspeichermedien nach Anspruch 5, wobei der eine oder die mehreren vordefinierten Impulsparameter mindestens eines von einer Impulsdauer, einem Impulstyp und einer angelegten Spannung umfassen.

## Revendications

**1.** Procédé mis en oeuvre par processeur, comprenant :

l'obtention (202), via un ou plusieurs processeurs matériels, d'un modèle d'électroporation moléculaire d'une bicouche lipidique d'une couche de stratum corneum d'une peau humaine ;

l'application (204), via le ou les processeurs matériels, d'un champ électrique de polarisation à travers la bicouche lipidique qui forme un pore à l'intérieur de la bicouche lipidique de la couche de stratum corneum ;

la stabilisation (206), via le ou les processeurs matériels, du pore en réduisant le champ électrique appliqué et en obtenant un pore stable ;

l'introduction (208), via le ou les processeurs matériels, d'une ou plusieurs molécules de médicament sur la bicouche lipidique ayant le pore stable en présence d'un champ électrique stable ;

le calcul (210), à l'aide d'une technique de simulation de dynamique moléculaire, MD, exécutée par le ou les processeurs matériels, d'un coefficient de diffusion active et d'un coefficient de diffusion passive de la ou des molécules de médicament à travers le pore stable, dans lequel le coefficient de diffusion active fait référence à un coefficient de diffusion obtenu en présence d'un champ électrique et le coefficient de diffusion passive fait référence à un coefficient de diffusion obtenu en l'absence d'un champ électrique ;

la génération (212), via le ou les processeurs matériels, d'une structure macroscopique de peau de la couche de stratum corneum de la peau humaine, dans lequel la couche de stratum corneum de la peau humaine comprend des cornéocytes intégrés sous la forme d'une matrice lipidique ;

le calcul (214), à l'aide des coefficients de diffusion obtenus à l'aide du modèle d'électroporation moléculaire de la bicouche lipidique de la couche de stratum corneum de la peau humaine, via une technique d'analyse par éléments finis, FEA, exécutée par le ou les processeurs matériels, d'un gradient de concentration de la ou des molécules de médicament en présence du champ électrique de polarisation appliqué, dans lequel le calcul du gradient de concentration comprend la résolution d'une équation de Laplace et d'une équation de Nernst-Planck dans des régions lipidiques de la couche de stratum corneum respectivement, sur la base de -i- une ou plusieurs conditions limites prédéfinies de la structure macroscopique de la couche de stratum corneum, -ii- un ou plusieurs paramètres d'impulsion prédéfinis et -iii- une ou plusieurs propriétés obtenues à partir du modèle d'électroporation moléculaire,

dans lequel l'équation de Laplace est donnée par : $\nabla . (\sigma \nabla V) = 0$,
dans lequel l'équation de Nernst-Planck est donnée par :

$$\frac{\partial C}{\partial t} = \nabla . [(D\nabla C) + (mC\nabla V)],$$

où, $C$ = concentration de molécule de médicament perméante, $D$ = coefficient de diffusion active/passive de molécule de médicament dans la région lipidique de stratum corneum, $V$ = tension appliquée à travers la peau humaine, $\sigma$ = conductivité électrique et $m$ = mobilité électrophorétique, et

dans lequel la mobilité électrophorétique est calculée à l'aide d'une relation d'Einstein-Smoluchowski donnée par : $m = \frac{z*D*F}{R*T}$

où, $D$ est le coefficient de diffusion active/passive de la molécule de médicament dans le stratum corneum, $z$

est la charge de la molécule de médicament, *F* est la constante de Faraday, *R* est la constante de gaz et *T* est la température ; et

le calcul (216), via le ou les processeurs matériels, d'un profil de flux et d'un profil de libération cumulative de la ou des molécules de médicament à l'aide du gradient de concentration obtenu à l'aide de la technique FEA ; l'ajustement du ou des paramètres d'impulsion prédéfinis sur la base du profil de flux calculé et du profil de libération cumulative calculé, à l'aide d'une simulation de conception factorielle complète pour sélectionner un protocole d'électroporation pour obtenir un profil de libération souhaité pour la ou les molécules de médicament, dans lequel le protocole d'électroporation consiste à ajuster le ou les paramètres d'impulsion prédéfinis pour obtenir le profil de libération souhaité pour la ou les molécules de médicament.

2. Procédé mis en oeuvre par processeur selon la revendication 1, dans lequel le ou les paramètres d'impulsion prédéfinis comprennent au moins l'un d'une durée d'impulsion, d'un type d'impulsion et d'une tension appliquée.

3. Système (100), comprenant :

une mémoire (102) stockant des instructions ;
une ou plusieurs interfaces de communication (106) ; et
un ou plusieurs processeurs matériels (104) couplés à la mémoire (102) via la ou les interfaces de communication (106), dans lequel le ou les processeurs matériels (104) sont configurés par les instructions pour :

obtenir un modèle d'électroporation moléculaire d'une bicouche lipidique d'une couche de stratum corneum d'une peau humaine ;
appliquer un champ électrique à travers la bicouche lipidique qui forme un pore à l'intérieur de la bicouche lipidique de la couche de stratum corneum ;
stabiliser le pore pour obtenir un pore stable en réduisant le champ électrique appliqué et obtenir un pore stable ;
introduire une ou plusieurs molécules de médicament sur la bicouche lipidique ayant le pore stable en présence du champ électrique ;
calculer, à l'aide d'une technique de simulation de dynamique moléculaire, MD, un coefficient de diffusion active et un coefficient de diffusion passive de la ou des molécules de médicament à travers le pore stable, dans lequel le coefficient de diffusion active fait référence à un coefficient de diffusion obtenu en présence d'un champ électrique et le coefficient de diffusion passive fait référence à un coefficient de diffusion obtenu en l'absence d'un champ électrique ;
générer une structure macroscopique de la couche de stratum corneum de la peau humaine, dans lequel la couche de stratum corneum de la peau humaine comprend des cornéocytes intégrés dans une matrice lipidique ; calculer, à l'aide des coefficients de diffusion obtenus à l'aide du modèle d'électroporation moléculaire de la bicouche lipidique de la couche de stratum corneum de la peau humaine, via une technique d'analyse par éléments finis, FEA, un gradient de concentration de la ou des molécules de médicament en présence du champ électrique, dans lequel le gradient de concentration est calculé en résolvant une équation de Laplace et une équation de Nernst-Planck dans des régions lipidiques de la couche de stratum corneum respectivement, sur la base de -i-une ou plusieurs conditions limites prédéfinies de la structure macroscopique de la couche de stratum corneum, -ii- un ou plusieurs paramètres d'impulsion prédéfinis et -iii- une ou plusieurs propriétés obtenues à partir du modèle d'électroporation moléculaire,
dans lequel l'équation de Laplace est donnée par : $\nabla . (\sigma \nabla V) = 0$,
dans lequel l'équation de Nernst-Planck est donnée par :

$$\frac{\partial C}{\partial t} = \nabla . [(D \nabla C) + (m C \nabla V)],$$

où, *C* = concentration de molécule de médicament perméante, *D* = coefficient de diffusion active/passive de molécule de médicament dans la région lipidique de stratum corneum, *V* = tension appliquée à travers la peau humaine, $\sigma$ = conductivité électrique et *m* = mobilité électrophorétique, et
dans lequel la mobilité électrophorétique est calculée à l'aide d'une relation d'Einstein-Smoluchowski donnée par : $m = \frac{z * D * F}{R * T}$

où, *D* est le coefficient de diffusion active/passive de la molécule de médicament dans le stratum corneum, *z* est la charge de la molécule de médicament, *F* est la constante de Faraday, *R* est la constante de gaz et *T* est la température ; et

calculer un profil de flux et un profil de libération cumulative de la ou des molécules de médicament à l'aide du gradient de concentration obtenu à l'aide de la technique FEA ;

ajuster le ou les paramètres d'impulsion prédéfinis sur la base du profil de flux calculé et du profil de libération cumulative calculé, à l'aide d'une simulation de conception factorielle complète pour sélectionner un protocole d'électroporation pour obtenir un profil de libération souhaité pour la ou les molécules de médicament, dans lequel le protocole d'électroporation consiste à ajuster le ou les paramètres d'impulsion prédéfinis pour obtenir le profil de libération souhaité pour la ou les molécules de médicament.

4.  Système (100) selon la revendication 3, dans lequel le ou les paramètres d'impulsion prédéfinis comprennent au moins l'un d'une durée d'impulsion, d'un type d'impulsion et d'une tension appliquée.

5.  Un ou plusieurs supports de stockage d'informations lisibles par machine non transitoires comprenant une ou plusieurs instructions qui, lorsqu'elles sont exécutées par un ou plusieurs processeurs matériels, provoquent la conception in silico d'expériences d'électroporation pour des applications d'administration de médicament topique et transdermique par :

l'obtention, via le ou les processeurs matériels, d'un modèle d'électroporation moléculaire d'une bicouche lipidique d'une couche de stratum corneum d'une peau humaine ;

l'application, via le ou les processeurs matériels, d'un champ électrique à travers la bicouche lipidique qui forme un pore à l'intérieur de la bicouche lipidique de la couche de stratum corneum ;

la stabilisation, via le ou les processeurs matériels, du pore en réduisant le champ électrique appliqué pour obtenir un pore stable ;

l'introduction, via le ou les processeurs matériels, d'une ou plusieurs molécules de médicament dans le pore stable en présence du champ électrique ;

le calcul, à l'aide d'une technique de simulation de dynamique moléculaire, MD, exécutée par le ou les processeurs matériels, d'un coefficient de diffusion active et d'un coefficient de diffusion passive de la ou des molécules de médicament sur la bicouche lipidique ayant le pore stable, dans lequel le coefficient de diffusion active fait référence à un coefficient de diffusion obtenu en présence d'un champ électrique et le coefficient de diffusion passive fait référence à un coefficient de diffusion obtenu en l'absence d'un champ électrique ;

la génération, via le ou les processeurs matériels, d'une structure macroscopique de peau de la couche de stratum corneum de la peau humaine, dans lequel la couche de stratum corneum de la peau humaine comprend des cornéocytes intégrés sous la forme d'une matrice lipidique ;

le calcul, à l'aide des coefficients de diffusion obtenus à l'aide du modèle d'électroporation moléculaire de la bicouche lipidique de la couche de stratum corneum de la peau humaine, via une technique d'analyse par éléments finis exécutée par le ou les processeurs matériels, d'un gradient de concentration de la ou des molécules de médicament en présence du champ électrique appliqué, dans lequel le calcul du gradient de concentration comprend la résolution d'une équation de Laplace et d'une équation de Nernst-Planck dans des régions lipidiques de la couche de stratum corneum respectivement, sur la base de -i- une ou plusieurs conditions limites prédéfinies de la structure macroscopique de la couche de stratum corneum, -ii- un ou plusieurs paramètres d'impulsion prédéfinis et -iii- une ou plusieurs propriétés obtenues à partir du modèle d'électroporation moléculaire,

dans lequel l'équation de Laplace est donnée par : $\nabla.\,(\sigma\nabla V) = 0$,

dans lequel l'équation de Nernst-Planck est donnée par :

$$\frac{\partial C}{\partial t} = \nabla.\,[(D\nabla C) + (mC\nabla V)],$$

où, $C$ = concentration de molécule de médicament perméante, $D$ = coefficient de diffusion active/passive de molécule de médicament dans la région lipidique de stratum corneum, $V$ = tension appliquée à travers la peau humaine, $\sigma$ = conductivité électrique et $m$ = mobilité électrophorétique, et

dans lequel la mobilité électrophorétique est calculée à l'aide d'une relation d'Einstein-Smoluchowski donnée par : $m = \dfrac{z*D*F}{R*T}$

où, $D$ est le coefficient de diffusion active/passive de la molécule de médicament dans le stratum corneum, $z$ est la charge de la molécule de médicament, $F$ est la constante de Faraday, $R$ est la constante de gaz et $T$ est la température ; et

le calcul, via le ou les processeurs matériels, d'un profil de flux et d'un profil de libération cumulative de la ou des molécules de médicament à l'aide du gradient de concentration obtenu à l'aide de la technique d'analyse par éléments finis ;

l'ajustement du ou des paramètres d'impulsion prédéfinis sur la base du profil de flux calculé et du profil de libération cumulative calculé, à l'aide d'une simulation de conception factorielle complète pour sélectionner un protocole d'électroporation pour obtenir un profil de libération souhaité pour la ou les molécules de médicament, dans lequel le protocole d'électroporation consiste à ajuster le ou les paramètres d'impulsion prédéfinis pour obtenir le profil de libération souhaité pour la ou les molécules de médicament.

6. Un ou plusieurs supports de stockage d'informations lisibles par machine non transitoires selon la revendication 5, dans lequel le ou les paramètres d'impulsion prédéfinis comprennent au moins l'un d'une durée d'impulsion, d'un type d'impulsion et d'une tension appliquée.

SYSTEM
100

MEMORY
102

DATABASE
108

HARDWARE
PROCESSOR(S)
104

INTERFACE(S)
106

**FIG. 1**

obtaining, via one or more hardware processors, a molecular electroporation model of lipid bilayer of stratum corneum layer of human skin — 202

applying an electric field across the lipid bilayer which forms a pore inside the lipid bilayer of the stratum corneum layer — 204

stabilizing the pore by reducing the applied electric field and obtaining a stable pore — 206

Introducing one or more drug molecules on the lipid bilayer having the stable pore in the presence of a stable electric field — 208

calculating, using a molecular dynamics (MD) simulation technique executed by the one or more hardware processors, one or more diffusion coefficients of the one or more drug molecules through the stable pore — 210

generating, a skin macroscopic structure of the stratum corneum layer of the human skin, wherein the stratum corneum layer of the human skin comprises corneocytes embedded in the form of a lipid matrix — 212

calculating, using the one or more diffusion coefficients obtained using the electroporation molecular model of the lipid bilayer of the stratum corneum of the human skin, via a finite element analysis (FEA) technique executed by the one or more hardware processors, concentration gradient of the one or more drug molecules in presence of electric potential — 214

calculating, via the one or more hardware processors, a flux profile and a cumulative release profile of the one or more drug molecules using the concentration gradient obtained using the FEA technique — 216

**FIG. 2**

FIG. 3

Pore Formation

Pore Growth

t= 0 ns  t= 6 ns  t= 6.2 ns  t= 6.4 ns

t= 6.8 ns  t= 7.0 ns  t= 7.2 ns  t= 8 ns

**FIG. 4**

FIG. 5

FIG. 6

FIG. 7A

FIG. 7B

FIG. 8A

FIG. 8B

**FIG. 9A**

**FIG. 9B**

FIG. 10A

FIG. 10B

FIG. 10C

FIG. 10D

FIG. 10E

FIG. 10F

FIG. 11A

FIG. 11B

FIG. 11C

FIG. 11D

FIG. 11E

FIG. 11F

FIG. 12A

FIG. 12B

FIG. 12C

FIG. 12D

FIG. 13A

FIG. 13B

**FIG. 14**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201721007631

**Non-patent literature cited in the description**

- **GAJULA KISHORE et al.** A Multiscale Simulation Study of Drug Transport. *Silico Skin Model*
- **RAKESH GUPTA et al.** Electroporation of Skin Stratum Corneum Lipid Bilayer and Molecular Mechanism of Drug Transport. *A Molecular Dynamics Study*
- **S. M. BECKER et al.** A numerical study of the influence of chemically enhanced lower lipid phase transition temperatures. *Thermal in vivo skin electroporation pore development and charged macromolecule transdermal delivery*
- **VANBEVER et al.** Transdermal delivery of metoprolol by electroporation. *Pharm. Res.*, 1994, vol. 11, 1657-1662
- **PRAUSNITZ et al.** Transdermal transport efficiency during skin electroporation and iontophoresis. *J. Controlled Release*, 1996, vol. 38, 205-217
- **ZEWERT et al.** Creation of transdermal pathways for macromolecule transport by skin electroporation and a low toxicity, pathway-enlarging molecule. *Bioelectrochem. Bioenerg.*, 1999, vol. 49, 11-20
- **BECKER et al.** Thermal in vivo skin electroporation pore development and charged macromolecule transdermal delivery: a numerical study of the influence of chemically enhanced lower lipid phase transition temperatures. *Int. J. Heat Mass Transfer*, 2008, vol. 51, 2060-2074

- **PAVSELJ**. A numerical model of skin electropermeabilization based on in vivo experiments. *Ann. Biomed. Eng.*, 2007, vol. 35, 2138-2144
- **DERMOL-CERNE**. From cell to tissue properties-modeling skin electroporation with pore and local transport region formation. *IEEE Trans. Biomed. Eng.*, 2018, vol. 65, 458-468
- **HULCOVA et al.** Modelling and validation of dielectric properties of human skin in the MHz region focusing on skin layer morphology and material composition. *Journal of Physics D: Applied Physics*, vol. 45 (2), 025301
- **GAJULA et al.** In-Silico skin model: a multiscale simulation study of drug transport. *Journal of chemical information and modeling*, vol. 57 (8), 2027-2034
- **POTTS, GUY**. Predicting skin premeability. *Pharmaceutical research*, 1992, vol. 9 (5), 663-669
- **GUPTA et al.** Electroporation of Skin Stratum Corneum Lipid Bilayer and Molecular Mechanism of Drug Transport. *A Molecular Dynamic Study*, 2018, vol. 34, 5860-5870